(19)

Europäisches
Patentamt
European
Patent Office
Office européen
des brevets

(11) **EP 4 212 624 A1**

(12) **EUROPEAN PATENT APPLICATION**
published in accordance with Art. 153(4) EPC

(43) Date of publication:
**19.07.2023 Bulletin 2023/29**

(21) Application number: **21866017.3**

(22) Date of filing: **08.09.2021**

(51) International Patent Classification (IPC):
*C12N 15/12* (2006.01)      *A61K 38/19* (2006.01)
*A61K 49/14* (2006.01)      *C12N 15/85* (2006.01)
*C12N 15/87* (2006.01)

(52) Cooperative Patent Classification (CPC):
**A61K 38/19; A61K 45/00; A61K 49/14;**
**A61P 25/00; A61P 35/00; C07K 14/435;**
**C12N 5/06; C12N 15/85; C12N 15/87**

(86) International application number:
**PCT/CN2021/117302**

(87) International publication number:
**WO 2022/052964 (17.03.2022 Gazette 2022/11)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **08.09.2020   CN 202010934192**

(71) Applicant: **Neuragen Biotherapeutics (Suzhou)**
**Co., Ltd.**
**Suzhou, Jiangsu 215127 (CN)**

(72) Inventors:
• **LIU, Yueguang**
  **Shanghai 201203 (CN)**
• **CHEN, Rulei**
  **Shanghai 201203 (CN)**

(74) Representative: **Goddar, Heinz J.**
**Boehmert & Boehmert**
**Anwaltspartnerschaft mbB**
**Pettenkoferstrasse 22**
**80336 München (DE)**

Remarks:
The complete document including Reference
Table(s) and the Sequence Listing(s) can be
downloaded from the EPO website

(54) **FUNCTIONAL FRAGMENT FOR REPROGRAMMING, COMPOSITION, AND APPLICATION THEREOF**

(57)     Provided are a set of transcription factors capable of collaboratively promoting a glial cell to be transdifferentiated and reprogrammed to be a functional neuron or a similar neuron, and a transcription factor composition. By promoting the expression of the set of transcription factors in vivo or in vitro. the trans-differentiation of the glial cell can be effectively used to repair a nervous system damage or the trans-differentiation ability of a key transcription factor is used to limit the aggravation of a glial cell-derived brain tumor. Further provided is an application of the discovered transcription factor and a composition thereof in preparation of medication for a nervous system disease.

EP 4 212 624 A1

**Description**

**Background of the Invention**

Field of the Invention

The present invention belongs to the field of biotechnology and gene therapy. Specifically, the present invention relates to a method for transforming glial cell-derived cells into neurons, and a method for applying aforementioned method to repair nervous system damage or treat glial cell-derived tumors.

Description of Related Art

The main pathological changes caused by central nervous system injury and various neurodegenerative diseases in mammals are irreversible degeneration and necrosis of neurons and destruction of neural circuits. How to supplement and replace the dead and lost neurons in the injured and diseased brain or spinal cord, and reconstruct the neural circuit are the key steps of treatment. Because the self-repair ability of the central nervous system (brain and spinal cord) of adult mammals is very limited, it is difficult to make up for the loss of neurons by themselves.

Because the transplantation efficiency of exogenous neurons or neuro-derived cells is low, and there are potential risks of tumorigenicity and immunogenicity, in recent years, the emergence of cell reprogramming technology has brought revolutionary changes to regenerative medicine, and the induction of neurons by reprogramming astrocytes in vivo through the expression of single or combination of multiple transcription factors is expected to become an important new strategy of neuron replacement therapy.

Neuroglioma is referred to as glioma for short, also known as glioblastoma. It refers to all tumors of neuroepithelial origin in the broad sense, and tumors of various types of glial cells in the narrow sense. Glioma is one of the most lethal malignant tumors and the most common primary central nervous system tumor. It accounts for 30% of brain and central nervous system tumors and 80% of brain malignant brain tumors. It is a serious threat to human health. According to the classification scheme of the World Health Organization (WHO) in 1999, glioma is divided into astrocytoma, oligodendroglioma, ependymoma, mixed glioma, choroid plexus tumor, neuroepithelial histoma of uncertain origin, mixed neuroglial and neuroglial tumors, pineal parenchymal tumors, embryonal tumors, and neuroblastoma tumors. Glioma and normal nerve tissue grow in a crisscross manner, with unclear boundary. Tumor tissue is not easy to clean up and easy to relapse. At the same time, due to the existence of blood-brain barrier, common anti-tumor drugs have poor efficacy. At present, the treatment of glioma has not yet met the clinical needs of the medical community. In recent years, some studies have found that some neuro-derived transcription factors or combinations of transcription factors have been able to transform glioma cells into neuron-like cells in vitro or in vivo, and limit the proliferation of glioma cells. However, the existing transcription factors or combinations of transcription factors have only been proved to be used in vitro or in vivo with low conversion efficiency, which is difficult to achieve practical clinical application.

Therefore, finding suitable transcription factors or their combinations to induce glial cells to differentiate into active neurons in vivo is very important for the repair of brain and spinal cord nervous system. At the same time, using the reprogramming technology of transcription factors for reference to apply it to glial cell derived glioma is also a treatment plan that needs to be solved urgently at present.

**Summary of the Invention**

The present invention provides a group of transcription factors and transcription factor combinations that synergistically promote the trans-differentiation of glial cells and reprogram them into functional neurons or neuron-like cells. The invention also provides a method for increasing the expression of this group of transcription factors in vivo or in vitro, and the application of this group of transcription factors in the preparation of drugs for nervous system diseases.

The first aspect of the invention provides a set of functional fragments that can synergistically promote the trans-differentiation of glial cells, wherein the functional fragments contain at least one functional fragment that promotes the expression of transcription factors, selected from those that promote the expression of transcription factors such as NeuroD1, Brn2, Ascl1, Ngn2, Gsx1, Tbr1, Dlx2, Ptfla, Pax6 and/or Otx2.

In another preferred example, the trans-differentiation refers to the trans-differentiation or reprogramming of glial cells into functional neurons.

In another preferred example, the functional fragments promoting the expression of transcription factor at least include the functional fragment promoting the expression of Ascl1 transcription factor.

In another preferred example, the Ascl1 is an enhanced Ascl1, and its amino acid sequence is shown in SEQ ID No: 41.

In another preferred example, the functional fragments promoting the expression of transcription factor at least include the functional fragment promoting the expression of NeuroD1 transcription factor.

In another preferred example, the functional fragments promoting the expression of transcription factor at least include the functional fragment promoting the expression of Brn2 transcription factor.

In another preferred example, the functional fragments promoting the expression of the transcription factor at least include the functional fragment promoting the expression of the Ngn2 transcription factor.

In another preferred example, the functional fragments promoting the expression of transcription factor at least include the functional fragment promoting the expression of Gsx1 transcription factor.

In another preferred example, the functional fragments promoting the expression of transcription factor at least include the functional fragment promoting the expression of Tbr1 transcription factor.

In another preferred example, the functional fragments promoting the expression of transcription factor at least include the functional fragment promoting the expression of Dlx2 transcription factor.

In another preferred example, the functional fragments promoting the expression of transcription factor at least include the functional fragment promoting the expression of Ptf1a transcription factor.

In another preferred example, the functional fragments promoting the expression of transcription factor at least include the functional fragment promoting the expression of Pax6 transcription factor.

In another preferred example, the functional fragments promoting the expression of transcription factor at least include the functional fragment promoting the expression of Otx2 transcription factor.

In another preferred example, the functional fragments combination contains at least two functional fragments that promote the expression of transcription factors, which are selected from the functional fragments that promote the expression of transcription factors such as NeuroD1, Brn2, Ascl1, Ngn2, Gsx1, Tbr1, Dlx2, Ptfla, Pax6 and/or Otx2.

In another preferred example, the functional fragments promoting the expression of transcription factor at least include the functional fragment promoting the expression of Brn2 transcription factor and another functional fragment promoting the expression of transcription factor. The above another functional fragment promoting the expression of transcription factor is selected from any functional fragment that promotes the expression of NeuroD1, Ascl1, Ngn2, Gsx1, Tbr1, Dlx2, Ptfla, Pax6 or Otx2 and other transcription factors; More preferably, the above another functional fragment that promotes the expression of transcription factors is selected from any functional fragment that promotes the expression of transcription factors such as NeuroD1, Ascl1 or Ngn2.

In another preferred example, the functional fragments promoting the expression of transcription factors at least include the functional fragment promoting the expression of NeuroD1 transcription factor and another functional fragment promoting the expression of transcription factors. The above another functional fragment promoting the expression of transcription factors is selected from any functional fragment that promotes the expression of Brn2, Ascl1, Ngn2, Gsx1, Tbr1, Dlx2, Ptfla, Pax6 or Otx2 and other transcription factors; More preferably, the above another functional fragment that promotes the expression of transcription factors is selected from any functional fragment that promotes the expression of transcription factors such as Brn2, Ascl1 or Ngn2.

In another preferred example, the functional fragments promoting the expression of transcription factors at least include the functional fragment promoting the expression of Gsx1 transcription factor and another functional fragment promoting the expression of transcription factors. The above another functional fragment promoting the expression of transcription factors is selected from any functional fragment that promotes the expression of NeuroD1, Ascl1, Ngn2, Brn2, Tbr1, Dlx2, Ptf1a, Pax6 or Otx2 and other transcription factors; More preferably, the above another functional fragment that promotes the expression of transcription factors is selected from any functional fragment that promotes the expression of transcription factors such as Ascl1, Ngn2 or Tbr1.

In another preferred example, the functional fragments promoting the expression of transcription factors at least include the functional fragment promoting the expression of Tbr1 transcription factor and another functional fragment promoting the expression of transcription factors. The above another functional fragment promoting the expression of transcription factors is selected from any functional fragment that promotes the expression of NeuroD1, Ascl1, Ngn2, Brn2, Gsx1, Dlx2, Ptfla, Pax6 or Otx2 and other transcription factors; More preferably, the above another functional fragment that promotes the expression of transcription factors is selected from any functional fragment that promotes the expression of transcription factors such as Ascl1, Ngn2 or Gsx1.

In another preferred example, the functional fragments promoting the expression of transcription factors at least include the functional fragment promoting the expression of Dlx2 transcription factor and another functional fragment promoting the expression of transcription factors. The above another functional fragment promoting the expression of transcription factors is selected from any functional fragment that promotes the expression of NeuroD1, Ascl1, Ngn2, Brn2, Tbr1, Gsx1, Ptfla, Pax6 or Otx2 and other transcription factors; More preferably, the above another functional fragment that promotes the expression of transcription factors is selected from any functional fragment that promotes the expression of transcription factors such as Ascl1, Ngn2 or Ptf1a.

In another preferred example, the functional fragments promoting the expression of transcription factors at least include the functional fragment promoting the expression of Ptf1a transcription factor and another functional fragment promoting the expression of transcription factors. The above another functional fragment promoting the expression of transcription factors is selected from any functional fragment that promotes the expression of NeuroD1, Ascl1, Ngn2, Brn2, Tbr1, Gsx1, Dlx2, Pax6 or Otx2 and other transcription factors; More preferably, the above another functional fragment that promotes the expression of transcription factors is selected from any functional fragment that promotes the expression of transcription factors such as Ascl1, Ngn2 or Dlx2.

In another preferred example, the functional fragments promoting the expression of transcription factors at least include the functional fragment promoting the expression of Pax6 transcription factor and another functional fragment promoting the expression of transcription factors. The above another functional fragment promoting the expression of transcription factors is selected from any functional fragment that promotes the expression of NeuroD1, Ascl1, Ngn2, Brn2, Tbr1, Gsx1, Ptfla, Dlx2 or Otx2 and other transcription factors; More preferably, the above another functional fragment that promotes the expression of transcription factors is selected from any functional fragment that promotes the expression of transcription factors such as Ascl1, Ngn2 or Otx2.

In another preferred example, the functional fragments promoting the expression of transcription factors at least include the functional fragment promoting the expression of Otx2 transcription factor and another functional fragment promoting the expression of transcription factors. The above another functional fragment promoting the expression of transcription factors is selected from any functional fragment that promotes the expression of NeuroD1, Ascl1, Ngn2, Brn2, Tbr1, Gsx1, Ptfla, Dlx2 or Pax6 and other transcription factors; More preferably, the above another functional fragment that promotes the expression of transcription factors is selected from any functional fragment that promotes the expression of transcription factors such as Ascl1, Ngn2 or Pax6.

In another preferred example, the functional fragments that promote the expression of transcription factors at least include the functional fragments that promote the expression of any transcription factor of Ascl1 or Ngn2, and the other functional fragments that promote the expression of transcription factors, which are selected from any functional fragments that promote the expression of transcription factors such as NeuroD1, Brn2, Gsx1, Tbr1, Dlx2, Ptf1a, Pax6 or Otx2.

In another preferred example, the functional fragments that can synergistically promote the trans-differentiation of glial cells at least include the functional fragments that promote the expression of the two transcription factors NeuroD1 and Brn2, or the functional fragments that promote the expression of the two transcription factors Gsx1 and Tbr1, or the functional fragments that promote the expression of the two transcription factors Dlx2 and Ptfla, or the functional fragments that promote the expression of the two transcription factors Pax6 and Otx2.

In another preferred example, the functional fragments that can synergistically promote the trans-differentiation of glial cells at least includes the combination of the functional fragments that can promote the expression of any transcription factor of Ascl1 or Ngn2 and another functional fragments that can synergistically promote the trans-differentiation of glial cells. The above another functional fragments that can synergistically promote the trans-differentiation of glial cells are selected from the combination of functional fragments that promote the expression of NeuroD 1 and Brn2 transcription factors, or the combination of functional fragments that promote the expression of Gsx1 and Tbr1 transcription factors, or the combination of functional fragments that promote the expression of Dlx2 and Ptf1a transcription factors, or the combination of functional fragments that promote the expression of Pax6 and Otx2 transcription factors.

The functional fragments that can synergistically promote the trans-differentiation of glial cells or the expression of transcription factors can be polynucleotides that encode the transcription factors, or functional proteins and peptides that are translated from polynucleotides, or small molecular drugs, macromolecular drugs, nucleic acid drugs that promote the expression of transcription factors, or polynucleotides or functional proteins that are located upstream of the transcription factors and can regulate the up-regulation of the expression of transcription factors, Peptides, small molecule drugs or macromolecular drugs, nucleic acid drugs, etc.

In another preferred example, the functional fragments that can synergistically promote the trans-differentiation of glial cells or the functional segment that can promote the expression of transcription factors are the functional protein of NeuroD1, Brn2, Ascl1, Ngn2, Gsx1, Tbr1, Dlx2, Ptf1a, Pax6 and/or Otx2 or the nucleic acid sequence encoding the functional protein of transcription factors such as NeuroD1, Brn2, Ascl1, Ngn2, Gsx1, Tbr1, Dlx2, Ptf1a, Pax6 and/or Otx2; Preferably, the functional fragments that can synergistically promote the trans-differentiation of glial cells or promote the expression of transcription factors are derived from mammals; Further preferably, from human or non-human primate mammals.

In another preferred example, the combination of the functional fragments is selected from the following group:

    (Z1) NeuroD1+Brn2;
    (Z2) Ascl1+Ngn2;
    (Z3) Ngn2+NeuroD1;
    (Z4) Gsx1+Tbr1;
    (Z5) Dlx2+Ptfla;
    (Z6) Pax6+Otx2;
    (Zn) The combination of (Z1) to (Z6) above.

In another preferred example, the combination of the functional fragments is selected from the following group: NeuroD1+Brn2; Gsx1+Tbr1;Dlx2+Ptfla; Pax6+Otx2; Or a combination thereof.

In another preferred example, the functional fragments that can synergistically promote the trans-differentiation of glial cells or the functional fragment that can promote the expression of transcription factors are a functional NeuroD1 protein, and the protein sequence is SEQ ID NO.: 1 or SEQ ID NO.: 2; The polynucleotide sequence encoding the NeuroD1 functional protein is shown in SEQ ID NO.: 3 or SEQ ID NO.: 4.

In another preferred example, the functional fragments that can synergistically promote the trans-differentiation of glial cells or the functional fragment that can promote the expression of transcription factors are a functional Brn2 protein, and the protein sequence is SEQ ID NO.: 5 or SEQ ID NO.: 6; The polynucleotide sequence encoding the Brn2 functional protein is shown in SEQ ID NO.: 7 or SEQ ID NO.: 8.

In another preferred example, the functional fragments that can synergistically promote the trans-differentiation of glial cells or the functional fragment that can promote the expression of transcription factors are a functional Ascl1 protein, and the protein sequence is SEQ ID NO.: 9 or SEQ ID NO.: 10 or SEQ ID NO.: 41; The polynucleotide sequence encoding the Ascl1 functional protein is shown in SEQ ID NO.: 11 or SEQ ID NO.: 12.

In another preferred example, the functional fragments that can synergistically promote the trans-differentiation of glial cells or the functional fragment that can promote the expression of transcription factors are a functional Ngn2 protein, and the protein sequence is SEQ ID NO.: 13 or SEQ ID NO.: 14; The polynucleotide sequence encoding the Ngn2 functional protein is shown in SEQ ID NO.: 15 or SEQ ID NO.: 16.

In another preferred example, the functional fragments that can synergistically promote the trans-differentiation of glial cells or the functional fragment that can promote the expression of transcription factors are a functional Gsx1 protein, and the protein sequence is SEQ ID NO.: 17 or SEQ ID NO.: 18; The polynucleotide sequence encoding the Gsx1 functional protein is shown in SEQ ID NO.: 19 or SEQ ID NO.: 20.

In another preferred example, the functional fragments that can synergistically promote the trans-differentiation of glial cells or the functional fragment that can promote the expression of transcription factors are a functional Tbr1 protein, and the protein sequence is SEQ ID NO.: 21 or SEQ ID NO.: 22; The polynucleotide sequence encoding the Tbr1 functional protein is shown in SEQ ID NO.: 23 or SEQ ID NO.: 24.

In another preferred example, the functional fragments that can synergistically promote the trans-differentiation of glial cells or the functional fragment that can promote the expression of transcription factors are a functional Dlx2 protein, and the protein sequence is SEQ ID NO.: 25 or SEQ ID NO.: 26; The polynucleotide sequence encoding the Dlx2 functional protein is shown in SEQ ID NO.: 27 or SEQ ID NO.: 28.

In another preferred example, the functional fragments that can synergistically promote the trans-differentiation of glial cells or the functional fragment that can promote the expression of transcription factors are a functional Ptf1a protein, and the protein sequence is SEQ ID NO.: 29 or SEQ ID NO.: 30; The polynucleotide sequence encoding the Ptf1a functional protein is shown in SEQ ID NO.: 31 or SEQ ID NO.: 32.

In another preferred example, the functional fragments that can synergistically promote the trans-differentiation of glial cells or the functional fragment that can promote the expression of transcription factors are a functional Pax6 protein, and the protein sequence is SEQ ID NO.: 33 or SEQ ID NO.: 34; The polynucleotide sequence encoding the Pax6 functional protein is shown in SEQ ID NO.: 35 or SEQ ID NO.: 36.

In another preferred example, the functional fragments that can synergistically promote the trans-differentiation of glial cells or the functional fragment that can promote the expression of transcription factors are a functional Otx2 protein, and the protein sequence is SEQ ID NO.: 37 or SEQ ID NO.: 38; The polynucleotide sequence encoding the Otx2 functional protein is shown in SEQ ID NO.: 39 or SEQ ID NO.: 40.

In another preferred example, the functional fragments that can synergistically promote the trans-differentiation of glial cells or the functional fragments that can promote the expression of transcription factors are the modified Ascl1 functional protein, and the protein sequence is shown in SEQ ID NO.: 41.

In another preferred example, when the functional fragments that can synergistically promote the trans-differentiation of glial cells or the functional segment that can promote the expression of transcription factors are functional protein, the sequence of the functional protein and SEQ ID NO.: 1, 2, 5, 6, 9, 10, 13, 14, 17, 18, 21, 22, 25, 26, 29, 30, 33, 34, 37, 38 and/or 41 have no less than 85% homology; More preferably, the sequence of the functional protein and SEQ ID NO.: 1, 2, 5, 6, 9, 10, 13, 14, 17, 18, 21, 22, 25, 26, 29, 30, 33, 34, 37, 38 and/or 41 sequence have no less than 95% homology; Preferably, the sequence homology of the functional protein with SEQ ID NO.: 1, 2, 5, 6, 9, 10, 13, 14, 17, 18, 21, 22, 25, 26, 29, 30, 33, 34, 37, 38 and/or 41 is not less than 99%.

In another preferred example, when the functional fragments that can synergistically promote the trans-differentiation of glial cells or the functional fragments that can promote the expression of transcription factors are the polynucleotide that encodes the functional protein, the sequence of the poly-nucleic acid that encodes the functional protein and SEQ ID NO.: 3, 4, 7, 8, 11, 12, 15, 16, 19, 20, 23, 24, 27, 28, 31, 32, 35, 36, 39 and/or 40 have a sequence homology of not less than 75%; More preferably, the sequence of the poly-nucleic acid encoding the functional protein and SEQ ID NO.: 3, 4, 7, 8, 11, 12, 15, 16, 19, 20, 23, 24, 27, 28, 31, 32, 35, 36, 39 and/or 40 have no less than 85% homology;

Preferably, the sequence of the poly-nucleic acid encoding the functional protein and SEQ ID NO.: 3, 4, 7, 8, 11, 12, 15, 16, 19, 20, 23, 24, 27, 28, 31, 32, 35, 36, 39 and/or 40 have no less than 95% homology.

Preferably, the glial cells are any astrocytes, NG2 glial cells, oligodendrocytes, microglial cells, or glial cells in injured state, tumor cells derived from glial cells, etc. from human or non-human mammals; The glial cells in the injured state are glial cells in the state that the tissue or the surrounding environment of glial cells is in the state of mechanical trauma, stroke or neurodegenerative disease causing neuron death and apoptosis, which leads to the blockage or disorder of nerve signal transmission; The tumor cells derived from the glial cells are generally glioma cells, which are selected from astrocytoma, oligodendroglioma, ependymoma, mixed glioma, choroid plexus tumor, neuroepithelial histoma of uncertain origin, mixed tumor of neurons and neuroglia, pineal parenchyma tumor, embryonal tumor and neuroblastoma tumor derived from human or non-human mammals.

Preferably, the functional nerve cell or neuroid cell comprises at least one of the following features:

(1) Similar to the morphology of nerve cells;
(2) The level of nerve cell-specific gene expression was up-regulated; The up-regulated genes include one or more of DCX, Tuj 1, Map2, NeuN, Synapsin I (antibody to synaptophysin 1);
(3) The level of glial cell-specific gene expression was down regulated; The downregulated genes include One or more of GFAP, S100 β, Glast and Acsbg1;
(4) Specific electrophysiological characteristics of nerve cells; That is, the cells have resting potential and action potential induced by the action of excitatory neurotransmitter or inhibitory neurotransmitter; The generated cell resting potential is not higher than - 50 mV, preferably, the generated cell resting potential is not higher than - 55 mV, or not higher than - 60 mV, or not higher than - 65 mV; The excitatory neurotransmitter includes but is not limited to glutamate or kainate, which can induce inward current; The inhibitory neurotransmitter includes but is not limited to glycine or γ-aminobutyric acid (GABA) can induce outward current;
(5) Forming functional synapses; The synapse can receive excitatory or inhibitory signal input and output action potential.

The second aspect of the invention provides a method for promoting the trans-differentiation and reprogramming of glial cells into functional neurons or neuron-like cells.

In another preferred example, the method is non-therapeutic and non-diagnostic.

In another preferred example, the method is in vitro.

In another preferred example, the method is therapeutic.

In another preferred example, the method includes the following steps: contact the functional fragments of the first aspect of the invention that can synergistically promote the gliocyte trans-differentiation with the gliocyte or rely on the delivery system to import, so as to make the gliocyte trans-differentiation and reprogramming into a functional neurons or neuron-like cells.

Preferably, the glial cells are any astrocytes, NG2 glia, oligodendrocytes, microglia, or glia in a damaged state, tumor cells derived from glia, etc. from human or non-human mammals; The glial cells in the injured state are glial cells in the state that the tissue or the surrounding environment of glial cells is in the state of mechanical trauma, stroke or neurodegenerative disease causing neuron death and apoptosis, which leads to the blockage or disorder of nerve signal transmission; The tumor cells derived from the glial cells are generally glioma cells, which are selected from astrocytoma, oligodendroglioma, ependymoma, mixed glioma, choroid plexus tumor, neuroepithelial histiocoma of uncertain origin, mixed tumor of neurons and neuroglia, pineal parenchyma tumor, embryonal tumor and neuroblastoma tumor derived from human or non-human mammals.

Any method of increasing the expression of transcription factors that can promote the trans-differentiation of glial cells, including but not limited to increasing the expression of any NeuroD1, Brn2, Ascl1, Ngn2, Gsx1, Tbr1, Dlx2, Ptfla, Pax6, Otx2 transcription factors in glial cells through direct contact or introduction with the glial cells, And promote the glial cells to display the characteristics of functional nerve cells or neuro-like cells.

The inducible factor or the functional fragment that promotes the expression of the transcription factor can be a polynucleotide encoding the transcription factor, or a functional protein or polypeptide after the translation of the polynucleotide, or a small molecule drug, a macromolecular drug, a nucleic acid drug that promotes the expression of any of the transcription factors NeuroD1, Brn2, Ascl1, Ngn2, Gsx1, Tbr1, Dlx2, Ptf1a, Pax6, Otx2, or polynucleotides or functional proteins, peptides, small molecule drugs or macromolecular drugs, nucleic acid drugs located in the upstream of any transcription factor of NeuroD1, Brn2, Ascl1, Ngn2, Gsx1, Tbr1, Dlx2, Ptfla, Pax6, Otx2 and regulating the up-regulation of transcription factor expression. The inducible factor or the functional fragment that promotes the expression of the transcription factor is passively absorbed by the glia or reaches the glia through the delivery system to take effect.

The delivery system includes, but is not limited to an expression vector containing functional fragments that promote the expression of transcription factors, nanoparticles wrapped with functional fragments that promote the expression of transcription factors, exosomes wrapped with functional fragments that promote the expression of transcription factors, viral vectors or cell vectors (such as modified red blood cells or bacteria) wrapped with functional fragments that promote the expression of transcription factors, and targeted effectors (such as glial cell specific antibody, polypeptide or other targeted substances) that contain functional fragments that promote the expression of transcription factors.

In another preferred example, the functional fragment of the inducer or the promoter of the expression of the transcription factor is a polynucleotide encoding the transcription factor, and the polynucleotides are selected from the transcription factor functional polynucleotides of NeuroD1, Brn2, Ascl1, Ngn2, Gsx1, Tbr1, Dlx2, Ptfla, Pax6 and/or Otx2; The polynucleotides need to be loaded in a viral or non-viral delivery system.

In another preferred example, the delivery system includes but is not limited to plasmids, viruses and cell vectors; It is preferably a viral vector, including but not limited to adenovirus vector, adeno-associated virus vector (AAV), retrovirus expression vector or lentivirus vector, etc.

In another preferred example, the expression vector containing transcription factor polynucleotides also contains glial cell-specific promoters. The promoters include, but are not limited to, GFAP promoter, NG2 promoter, Aldh1L1 promoter, IBA1 promoter, CNP promoter, LCN2 promoter or promoter variants after genetic engineering.

In another preferred example, the promoter is GFAP promoter, or GFAP promoter after genetic engineering. Preferably, the human hGFAP promoter (SEQ ID No: 42) can be transformed into a truncated version of 683 bp (SEQ ID No: 43).

In another preferred example, the expression vector containing transcription factor polynucleotides also contains one or more regulatory elements that regulate gene expression, which are used to enhance gene expression level. The regulatory elements include but are not limited to CMV enhancer, SV40 enhancer, EN1 enhancer, VP16 fusion protein or enhancer variants after genetic engineering, as well as SV40 poly A tailing signal, human insulin gene poly A tailing signal or WPRE (regulatory elements after the transcription of marmot hepatitis B virus), human MAR sequence or variants after genetic engineering.

In another preferred example, the regulatory element used to enhance expression is the active domain (SEQ ID NO: 44) of VP16 protein from Herpes simplex virus, wherein the coding sequence (SEQ ID NO: 45) of VP16 can be loaded individually or in a string, and the fusion protein of VP16-transcription factor DNA binding region can be expressed through glial cell-specific promoter.

In another preferred example, the regulatory element used to enhance expression comes from the enhancer of simian vacuolating virus 40 SV40 (SEQ ID NO: 46). Inserting it into the glial cell-specific promoter can enhance the activity of the promoter and improve the efficiency of neuron induction.

In another preferred example, the expression vector containing transcription factor polynucleotides can also contain other functional fragments at the same time. The other functional fragments can be reporter genes or other transcription factor functional fragments with reprogramming function, including but not limited to selected from NeuroD1, Brn2, Ascl1, Ngn2, Gsx1, Tbr1, Dlx2, Ptfla, Pax6, Otx2, etc; Preferably, the same vector can contain polynucleotide fragments of at least two transcription factors, which can be expressed under one glial-specific promoter or under two glial-specific promoters respectively. When two or more transcription factors are in the transcript of a single promoter, the promoter is connected with the open reading frame of multiple transcription factors through multiple cis-transon elements. Among them, the transcription factors are separated by IRES or 2A polypeptide (P2A) elements to achieve the expression of multiple transcription factors (Pharmaceutics 2019, 11 (11), 580; The IRES sequence used in the invention is copied from Addgene # 69550; P2A sequence is copied from Addgene # 130692). The combination of the two transcription factors is selected from the combination of NeuroD1 and Brn2 transcription factors, the combination of Gsx1 and Tbr1 transcription factors, the combination of Dlx2 and Ptf1a transcription factors, the combination of Pax6 and Otx2 transcription factors, and the combination of Ascl1 and Ngn2 transcription factors. The molar concentration ratio of the expression of the two transcription factors is 4:1~1:4; Preferably, the molar concentration ratio of the expression amount of the two transcription factors is 2:1 to 1:2; Further preferably, the optimal molar concentration ratio of the expression of the two transcription factors is 1:1.

In another preferred example, the same vector contains at least two transcription factors, and one of them is Ascl1 or Ngn2. At this point, the molar concentration ratio of the expression amount of Ascl1 or Ngn2 is not less than 20%; Preferably, the molar concentration ratio of the expression amount of Ascl1 or Ngn2 is not less than 33%; Further preferably, the molar concentration ratio of the expression amount of Ascl1 or Ngn2 is not less than 50%; The vector includes any combination of the following transcription factors:

(1) The combination of Ascl1 and any other transcription factor, which is selected from NeuroD1, Brn2, Ascl1, Ngn2, Gsx1, Tbr1, Dlx2, Ptfla, Pax6 or Otx2;
(2) The combination of Ngn2 and any other transcription factor, which is selected from NeuroD1, Brn2, Ascl1, Ngn2, Gsx1, Tbr1, Dlx2, Ptfla, Pax6 or Otx2; and
(3) When carrying at least three transcription factors in the same vector, the transcription factors include at least the combination of Ascl1+NeuroD1+Brn2, Ascl1+Gsx1+Tbr1, Ascl1+Dlx2+Ptf1a, Ascl1+Pax6+Otx2 or Ngn2+NeuroD1+Brn2, Ngn2+Gsx1+Tbr1, Ngn2+Dlx2+Ptf1a, or Ngn2+Pax6+Otx2; Among them, except Ascl1 or Ngn2, the molar concentration ratio of the expression of the remaining two transcription factors is 4:1 to 1:4; Preferably, the molar concentration ratio of the expression amount of the two transcription factors is 2:1 to 1:2; Further preferably, the molar concentration ratio of the expression of the two transcription factors is 1:1.

In another preferred example, one or more expression vectors containing different transcription factor polynucleotides can also be used at the same time. The transcription factors are selected from the functional polynucleotides of the transcription factors of NeuroD1, Brn2, Ascl1, Ngn2, Gsx1, Tbr1, Dlx2, Ptf1a, Pax6 and/or Otx2; Preferably, the vector combination is selected from the vector combination containing the transcription factor NeuroD1 and the transcription factor Brn2, the vector combination containing the transcription factor Gsx1 and the transcription factor Tbr1, the vector combination containing the transcription factor Dlx2 and the transcription factor Ptf1a, and the vector combination containing the transcription factor Pax6 and the transcription factor Otx2. The molar concentration ratio of the expression of the two transcription factors is 4:1~1:4; Preferably, the molar concentration ratio of the expression amount of the two transcription factors is 2:1 to 1:2; Further preferably, the molar concentration ratio of the expression amount of the two transcription factors is 1:1.

In another preferred example, one or more expression vectors containing different transcription factor polynucleotides can also be used at the same time, including at least the combination of expression vectors containing Ascl1 or Ngn2 polynucleotides and other transcription factor vectors, wherein the molar concentration ratio of the expression amount of Ascl1 or Ngn2 should not be less than 20%, and preferably, the molar concentration ratio of the expression amount of Ascl 1 or Ngn2 should not be less than 33%; Further preferably, the molar concentration ratio of the expression amount of Ascl1 or Ngn2 should not be less than 50%; The expression vector is selected from any combination of the following vectors:

(1) The combination of the vector containing the transcription factor Ascl1 and another transcription factor, which is selected from NeuroD1, Brn2, Ascl1, Ngn2, Gsx1, Tbr1, Dlx2, Ptfla, Pax6 or Otx2;

(2) The vector containing the transcription factor Ngn2 is combined with the vector containing another transcription factor, which is selected from NeuroD1, Brn2, Ascl1, Ngn2, Gsx1, Tbr1, Dlx2, Ptf1a, Pax6 or Otx2;

(3) Vector combination containing transcription factors Ascl 1, NeuroD1 and Brn2, vector combination containing transcription factors Ascl1, Gsx1 and Tbr1, vector combination containing transcription factors Ascl 1, Dlx2 and Ptfla, vector combination containing transcription factors Ascl1, Pax6 and Otx2, or vector combination containing transcription factors Ngn2, NeuroD1 and Brn2, vector combination containing transcription factors Ngn2, Gsx1 and Tbr1, vector combination containing transcription factors Ngn2, Dlx2 and Ptf1a Vector combination containing transcription factors Ngn2, Pax6 and Otx2. Among them, except Ascl1 or Ngn2, the molar concentration ratio of the expression of the remaining two transcription factors is 4:1 to 1:4; Preferably, the molar concentration ratio of the expression amount of the two transcription factors is 2:1 to 1:2; Further preferably, the molar concentration ratio of the expression amount of the two transcription factors is 1:1.

In another preferred example, the expression vector of the functional fragment containing the transcription factor polynucleotide is a lentivirus vector; Lentiviral vector contains viral ITR sequence, CAG promoter, coding frame of functional fragment of transcription factor polynucleotide, post transcriptional regulatory element WPRE, etc; The expression vector can also contain a reporter gene, but the reporter gene is not necessary in practical application. The lentiviral vector from 5 'to 3' ends can successively include the following elements: viral ITR sequence+CAG promoter+coding frame of transcription factor polynucleotide and green fluorescent protein GFP+post transcriptional regulatory element WPRE+viral ITR sequence+promoter and coding frame of ampicillin resistance gene. Among them, the coding frame of GFP and the promoter and coding frame of ampicillin resistance gene are not necessary. Preferably, the polynucleotides of the transcription factors are selected from the functional polynucleotides encoding NeuroD1, Brn2, Ascl1, Ngn2, Gsx1, Tbr1, Dlx2, Ptfla, Pax6 and/or Otx2; Specifically, from the sequence of SEQ ID NO.: 3, 4, 7, 8, 11, 12, 15, 16, 19, 20, 23, 24, 27, 28, 31, 32, 35, 36, 39 and/or 40.

In another preferred example, the expression vector of the functional fragment containing the transcription factor polynucleotide is GFAP-AAV vector; GFAP-AAV vector contains viral ITR sequence, CMV enhancer, human GFAP promoter, coding frame of functional fragment of transcription factor polynucleotide, post transcriptional regulatory element WPRE, etc; The expression vector can also contain a reporter gene, but the reporter gene is not necessary in practical application. The GFAP-AAV expression vector can successively include the following elements from the 5 'to 3' end: viral ITR sequence+CMV enhancer+human GFAP promoter+transcription factor polynucleotide and coding frame of red fluorescent protein mCherry+post transcriptional regulatory element WPRE+viral ITR sequence+promoter and coding frame of ampicillin resistance gene, wherein the coding frame of red fluorescent protein mCherry and the promoter and coding frame of ampicillin resistance gene are not necessary. Preferably, the polynucleotides of the transcription factors are selected from the functional polynucleotides encoding NeuroD1, Brn2, Ascl1, Ngn2, Gsx1, Tbr1, Dlx2, Ptfla, Pax6 and/or Otx2, specifically, from the sequence of SEQ ID NO.: 3, 4, 7, 8, 11, 12, 15, 16, 19, 20, 23, 24, 27, 28, 31, 32, 35, 36, 39 and/or 40.

In another preferred example, the GFAP-AAV expression vector can include the following elements from the 5 'to 3' ends: viral ITR sequence+SV40 enhancer+human GFAP promoter+transcription factor polynucleotide+post transcriptional regulatory element WPRE+viral ITR sequence.

In another preferred example, the GFAP-AAV expression vector can include the following elements from the 5 'to 3' end in turn: viral ITR sequence+CMV enhancer+human GFAP promoter+VP16 fusion protein+DNA binding region of transcription factor+post transcriptional regulatory element WPRE+viral ITR sequence.

In another preferred example, the GFAP-AAV expression vector can include the following elements from the 5 'to 3' ends: viral ITR sequence+CMV enhancer+human truncated GFAP promoter+transcription factor polynucleotide+post transcriptional regulatory element WPRE+viral ITR sequence.

In another preferred example, the GFAP-AAV expression vector from the 5 'to the 3' end can successively include the following elements: viral ITR sequence+enhancer of SV40+promoter of human truncated GFAP+VP16 fusion protein+DNA binding region of transcription factor+post transcriptional regulatory element WPRE+viral ITR sequence.

In another preferred example, the GFAP-AAV expression vector can include the following elements from the 5 'to 3' ends: viral ITR sequence+SV40 enhancer+human truncated GFAP promoter+transcription factor polynucleotide+post transcriptional regulatory element WPRE+viral ITR sequence.

In another preferred example, the GFAP-AAV expression vector can include the following elements from the 5 'to 3' end in turn: viral ITR sequence+enhancer of SV40+promoter of human GFAP+VP16 fusion protein+DNA binding region of transcription factor+post transcriptional regulatory element WPRE+viral ITR sequence.

In another preferred example, the GFAP-AAV expression vector can include the following elements from the 5 'to 3' ends in turn: viral ITR sequence+CMV enhancer+human truncated GFAP promoter+VP16 fusion protein+DNA binding region of transcription factors+post transcriptional regulatory element WPRE+viral ITR sequence.

The third aspect of the invention provides a pharmaceutical composition, which comprises:

(A) The functional fragments promoting the expression of transcription factors described in the first aspect of the invention; And/or

(B) Functional neurons or neuron-like cells, which are obtained by promoting glial cell trans-differentiation and reprogramming by the method described in the second aspect of the invention; and

(C) Pharmaceutical acceptable excipients.

In another preferred example, the pharmaceutical composition is a liquid preparation and a lyophilized preparation.

In another preferred example, the pharmaceutical composition is an injection.

In another preferred example, the pharmaceutical composition comprises:

(A) The combination of functional fragments that promote the expression of transcription factors, or functional neurons or neuron-like cells, which are obtained by processing glial cells with the combination of the functional fragments and making them trans-differentiation and reprogramming;

Wherein, the combination contains Ascl1 or enhanced Ascl1; Or Ngn2; or

The combination described is selected from the following group:

(Z1) NeuroD1+Brn2;
(Z2) Ascl1+Ngn2;
(Z3) Ngn2+NeuroD1;
(Z4) Gsx1+Tbr1;
(Z5) Dlx2+Ptfla;
(Z6) Pax6+Otx2; and
(Zn) the combination of (Z1) to (Z6) above;

(B) Pharmaceutical acceptable excipients.

In another preferred example, the above combination is the combination of enhanced Ascl1 and at least one selected from the following group: NeuroD1, Brn2, Ngn2, Gsx1, Tbr1, Dlx2, Ptf1a, Pax6, Otx2.

In another preferred example, the above combination is a combination of Ascl1 and at least one selected from the following group: NeuroD1, Brn2, Ngn2, Gsx1, Tbr1, Dlx2, Ptfla, Pax6, Otx2.

In another preferred example, the above combination is a combination of Ngn2 and at least one selected from the following group: NeuroD1, Brn2, Ngn2, Gsx1, Tbr1, Dlx2, Ptfla, Pax6, Otx2.

The fourth aspect of the invention provides an artificial reprogrammed neuron or neuron-like cell, which is obtained from glial cells through trans-differentiation and reprogramming.

In another preferred example, the artificial reprogrammed neuron or neuron-like is prepared by the method described in the second aspect of the invention.

The fifth aspect of the present invention provides the use of the pharmaceutical composition described in the third aspect of the present invention or the artificial reprogrammed neurons or neuron-like cells in the fourth aspect, that is, they are used to prepare drugs for gene therapy of nervous system diseases. Preferably, the nervous system disease is a nervous system injury or a glioma derived from glial cells.

It should be understood that within the scope of the invention, the above technical features of the invention and the technical features specifically described in the following (according to the embodiment) can be combined with each other to form a new or preferred technical solution. Limited by space, I will not repeat it here.

**Brief Description of the Figures**

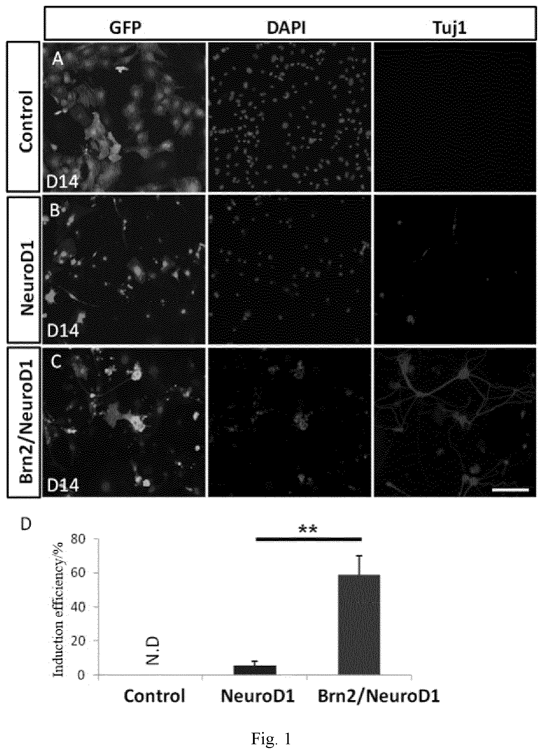

Fig. 1 shows that the combination of Brn2 and NeuroD 1 can induce human glioma cells into neurons. Figure 1A-C shows the expression of Tuj 1, a marker of neuronal characteristics, by immunofluorescence after 14 days of infection of human glioma cell U251 cells with control lentivirus FUGW, single virus FUGW - NeuroD1, and the combination of two viruses FUGW - Brn2 and FUGW - NeuroD1. Figure 1D shows the statistical diagram of the ratio of different virus-induced neurons. "* *" stands for p<0.01. Scale: 50 $\mu$m.

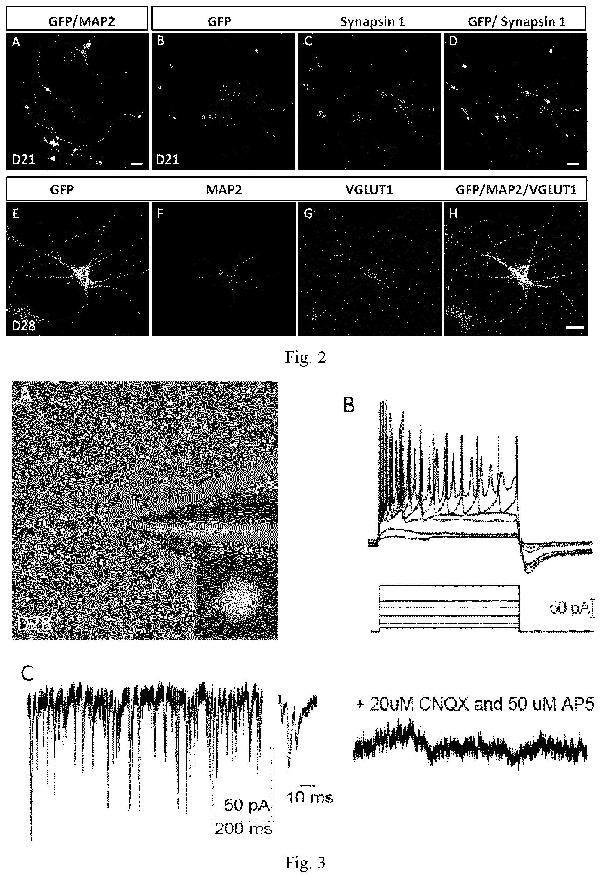

Fig. 2 shows the molecular expression properties of neurons induced by the combination of Brn2 and NeuroD1. Figure 2A shows that 21 days after glioma cells were infected with lentivirus, the induced neurons expressed the marker molecule MAP2 of mature neurons. Figure 2B-D shows that the induced neurons express the marker molecule Synapsin I of mature neurons. Figure 2E-H shows the transmitter properties of the induced neurons, which express the glutamate neuron marker molecule VGLUT1. Scale: 20 $\mu$m.

Fig. 3 shows the electrophysiological properties of neurons induced by the combination of Brn2 and NeuroD1. Figure 3A shows the cells being recorded by the glass electrode (with green fluorescence). Figure 3B shows that the induced neurons can emit action potentials. Figure 3C shows that the postsynaptic current signal of the induced neuron is detected, and the postsynaptic current signal disappears after adding blockers CNQX and AP5.

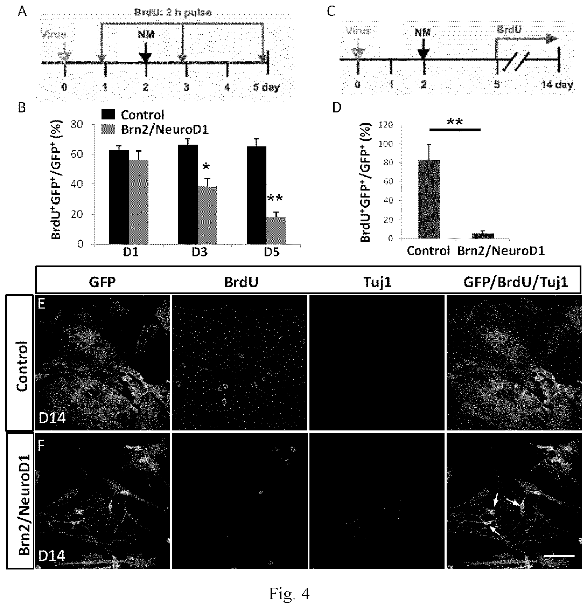

Fig. 4 shows that the combination of Brn2 and NeuroD1 induces neurons to exit the cell cycle. Figure 4A and B show that the BrdU positive number of cells induced by the combination of Brn2/NeuroD1 decreased sharply when the BrdU interval was labeled at different time periods of virus infection. Figure 4C-F shows that the number of BrdU positive cells induced by the combination of Brn2/NeuroD1 decreased significantly after 5 days of virus infection when BrdU was continuously incorporated into the label. The arrow in Fig. 4F indicates that the induced neurons are BrdU negative. "*" stands for p<0.05. "* *" stands for p<0.01. Scale: 50 $\mu$m.

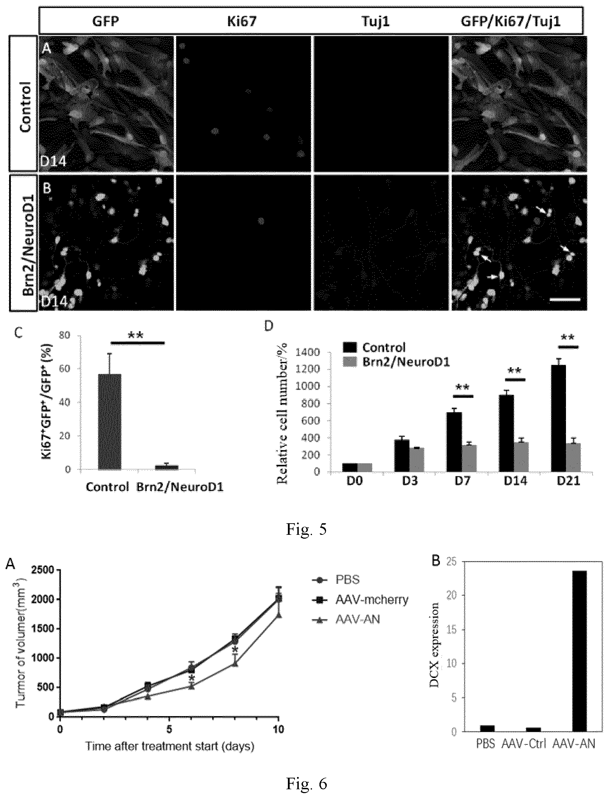

Fig. 5 shows that the combination of Brn2 and NeuroD1 inhibits the growth of glioma cells. Figure 5A-C shows that after 14 days of virus infection, immunocytochemical analysis of Ki67 shows that the number of Ki67 positive cells in the combination of Brn2/NeuroD1 is significantly reduced. The arrow in Figure 5B indicates that the induced neurons are Ki67 negative. Figure 5D shows the number of cells counted after different time of virus infection. "* *" stands for p<0.01. Scale: 50 $\mu$m.

Fig. 6 shows that the AAV vector expressing reprogramming factors NeuroD 1 and Brn2 inhibits the growth of glioma in animals by inducing transdifferentiated neurons. Figure 6A shows the tumor volume at each time point in different administration groups, and Figure 6B shows the results of Real-time PCR analysis of tumor samples in different administration groups. "*" stands for p<0.05.

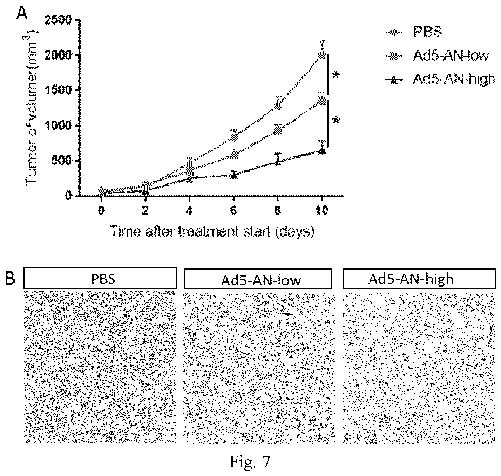

Fig. 7 shows that adenovirus type 5 expressing reprogramming factors NeuroD1 and Brn2 inhibits glioma growth in animals by inducing trans-differentiation of neurons. Figure 7A shows the tumor volume at each time point in different administration groups, and Figure 7B shows the HE color results of tumor samples in different administration groups. "*" stands for p<0.05.

**Detailed Description of the Invention**

After extensive and in-depth research, the inventor unexpectedly discovered a number of transcription factors or combinations of transcription factors with the function of trans-differentiation and reprogramming, the method of transforming glial cells into neurons, and the neurons which are able to efficiently convert glial cells into neuron cells with electrophysiological functions in vitro or in vivo. Based on such findings, the inventor further explored the application scenarios of transcription factors and their combinations. The combination of some specific transcription factors can synergistically significantly promote glial cells to differentiate into neurons. The method of the invention is applied to explore the application of nerve injury repair or brain glioma drug development, especially in the animal model of glioma, and it is observed that the reprogramming results in the withdrawal of glioma cells from the cell cycle, the tumor size of the animal is significantly reduced, and the survival time is significantly prolonged. Therefore, this batch of transcription factors or combinations of transcription factors with the function of trans-differentiation and reprogramming are expected to be used in the development of nerve injury repair drugs or glioma drugs.

Definitions

The term "administration" refers to the physical introduction of the product of the invention into the subject using any of the various methods and delivery systems known to those skilled in the art, including intravenous, intracerebral, intratumoral, intramuscular, subcutaneous, intraperitoneal, spinal cord or other parenteral administration routes, such as injection or infusion.

The term "about" may refer to a value or composition within the acceptable error range of a specific value or composition determined by a person skilled in the art, which will depend in part on how to measure or measure the value or composition. Generally, "about" means $\pm$ 10% or $\pm$ 20%. For example, about 1:1 means (1 $\pm$ 0.2): (1 $\pm$ 0.2); Or (1 $\pm$ 0.1): (1 $\pm$ 0.1).

As used in this article, the term "reprogramming" generally refers to the process of regulating or changing the biological activity of a cell and transforming it from one biological state to another, usually including differentiation (from progenitor cell to terminal cell), dedifferentiation (from terminal cell to pluripotent stem cell), trans-differentiation (from one terminal cell to another terminal cell), dedifferentiation (from terminal cell to progenitor cell) The process of changing the fate of cells, such as trans-differentiation (from one kind of progenitor cell to the terminal cell naturally differentiated from another kind of progenitor cell).

In the present invention, the "trans-differentiation" or "reprogramming" or "trans-differentiation reprogramming" specifically refers to the process from one terminal cell to another, specifically, to the process of transforming glial cells into functional nerve cells or neuro-like cells.

TRANSCRIPTION FACTOR

The present invention provides a group of transcription factors with reprogramming function. These transcription factors and their combinations have excellent trans-differentiation ability and can be used to promote the efficiency of glial cell trans-differentiation into neurons.

As used herein, the term "transcription factor of the present invention" refers to one or a group of transcription factors necessary for the differentiation of nerve cells selected from the following groups: NeuroD1, Brn2, Ascl1, Ngn2, Gsx1, Tbr1, Dlx2, Ptfla, Pax6 and Otx2. Preferably, the transcription factor of the present invention comprises at least two of the said transcription factors.

NeuroD1 functional fragment is a polynucleotide or its expressed protein fragment that is derived from mammals and encodes the transcription factor of Neurological differentiation 1. NeuroD1 is a bHLH (basic helix-loop-helix) transcription factor. For example, the ID # of NeuroD1 molecule from human is 4760 in GenBank, and its protein sequence is shown in SEQ ID NO.: 1; NCBI Reference Sequence: NM_ 002500.5, CDS sequence is shown in SEQ ID NO.: 3.

Brn2 functional fragment, also known as POU3F2, Oct7 or N-Oct3, is a polynucleotide or its expressed protein fragment encoding Pou class 3 homeobox 2 transcription factor derived from mammals. Brn2 is a family of neurocell-specific POU-III transcription factors. For example, Brn2 molecule from humans has ID # 5454 in GenBank, and its protein sequence is shown in SEQ ID NO.: 5; NCBI Reference Sequence: NM_ 005604.4, CDS sequence is shown in SEQ ID NO.: 7.

The functional fragment of Ascl1 is a polynucleotide or its expressed protein fragment encoding the transcription factor of Achaete-scute homolog 1 derived from mammals. Ascl1 is a bHLH (basic helix-loop-helix) transcription factor. For example, the ID # of Ascl1 molecule from human is 429 in GenBank, and its protein sequence is shown in SEQ ID NO.: 9; NCBI Reference Sequence: NM_ 004316.4, CDS sequence is shown in SEQ ID NO.: 11.

The functional fragment of Ngn2, also known as Neurog2, is a polynucleotide or its expressed protein fragment encoding Neurogenin-2 transcription factor derived from mammals. Ngn2 is a bHLH (basic helix-loop-helix) transcription factor. For example, the ID # of Ngn2 molecule from human is 63973 in GenBank, and its protein sequence is shown in SEQ ID NO.: 13; NCBI Reference Sequence: NM_ 024019.4, CDS sequence is shown in SEQ ID NO.: 15.

The functional fragment of Gsx1, also known as Gsh1, is a polynucleotide or its expressed protein fragment that is derived from mammals and encodes GS homeobox 1 transcription factor. The binding site of Gsx1 in DNA sequence is 5'- GC [TA] [AC] ATTA [GA] - 3'. For example, the ID # of Gsx1 molecule from human is 219409 in GenBank, and its egg white sequence is shown in SEQ ID NO.: 17; NCBI Reference Sequence: NM_ 145657.3, CDS sequence is shown in SEQ ID NO.: 19.

Tbr1 functional fragment is a polynucleotide or its expressed protein fragment that is derived from mammals and encodes T-box brain transcription factor 1 transcription factor. Tbr1 is a T-box transcription factor. For example, the ID # of Tbr1 molecule from human is 10716 in GenBank, and its protein sequence is shown in SEQ ID NO.: 21; NCBI Reference Sequence: NM_ 006593.4, CDS sequence is shown in SEQ ID NO.: 23.

Dlx2 functional fragment is a polynucleotide or its expressed protein fragment that is derived from mammalian and encodes the transcription factor of distal-less homeobox 2. Dlx2 is a transcription factor that participates in the terminal differentiation of intermediate neurons. For example, the ID # of Dlx2 molecule from human is 1746 in GenBank, and its protein sequence is shown in SEQ ID NO.: 25; NCBI Reference Sequence: NM_ 004405.4, CDS sequence is shown in SEQ ID NO.: 27.

Ptf1a functional fragment is a polynucleotide or its expressed protein fragment that is derived from mammals and encodes the transcription factor of pancreas-associated transcription factor 1a. Ptf1a is a transcription factor involved in pancreatic development. For example, the ID # of Ptf1a molecule from human is 256297 in GenBank, and its protein sequence is shown in SEQ ID NO.: 29; NCBI Reference Sequence: NM_ 178161.3, CDS sequence is shown in SEQ ID NO.: 31.

Pax6 functional fragment is a polynucleotide or its expressed protein fragment that is derived from mammalian and encodes the paired box 6 transcription factor. Pax6 is a key transcription factor involved in the development of neural tissue. For example, the ID # of Pax6 molecule from human is 5080 in GenBank, and its protein sequence is shown in SEQ ID NO.: 33; NCBI Reference Sequence: NM_ 000280.5, CDS sequence is shown in SEQ ID NO.: 35.

Otx2 functional fragment is a polynucleotide or its expressed protein fragment that is derived from mammalian and encodes the transcription factor of orthodentile homeobox 2. Otx2 belongs to the transcription factor of the subfamily of bicoid homologous domain. For example, the ID # of Otx2 molecule from human is 5015 in GenBank, and its protein sequence is shown in SEQ ID NO.: 37; NCBI Reference Sequence: NM_ 001270523.2, CDS sequence is shown in SEQ ID NO.: 39.

The present invention has no special restrictions on any method that can promote the expression of the functional fragments of the above transcription factors, including but not limited to promoting the expression or activity of any NeuroD1, Brn2, Ascl1, Ngn2, Gsx1, Tbr1, Dlx2, Ptf1a, Pax6, Otx2 transcription factors in the glial cells by direct contact or introduction of the inducible factor or the functional fragments that can promote the expression of the transcription factors, and promote the glial cells to display the characteristics of functional nerve cells or neuro-like cells; The inducible factor or the functional fragment that promotes the expression of the transcription factor can be a polynucleotide encoding the transcription factor, or a functional protein or polypeptide after the translation of the polynucleotide, or a small molecule drug, a macromolecular drug, a nucleic acid drug that promotes the expression of any of the transcription factors NeuroD1, Brn2, Ascl1, Ngn2, Gsx1, Tbr1, Dlx2, Ptfla, Pax6, Otx2, or polynucleotide or functional protein, polypeptide, small molecule drug or macromolecule drug located upstream of any transcription factor of NeuroD1, Brn2, Ascl1, Ngn2, Gsx1, Tbr1, Dlx2, Ptf1a, Pax6, Otx2. It is absorbed passively by glial cells or delivered to glial cells to take effect.

The method of promoting the expression of the functional fragments of the above transcription factors can also be obtained by CRISPR/dCas9 targeting the expression of the DNA-activated genes of the relevant transcription factors, or by CRISPR/Cas13 targeting the relevant transcription factor RNA to improve the expression of the functional proteins of the transcription factors.

Those skilled in the art can screen the promotion methods of the above transcription factors according to the existing database. It should be understood that, based on the function of the transcription factors disclosed in the present invention on the trans-differentiation of glial cells and the inhibition of neural injury repair and glioma cells, those skilled in the art can reasonably foresee that any substance that can promote the above transcription factors will have the function on the trans-differentiation of glial cells and the inhibition of neural injury repair and glioma cells.

Preferably, the reprogrammed transcription factor of the invention can be used in combination with the modified expression element to further improve the expression of the transcription factor of the invention.

GLIAL CELL

As used herein, the term " Neuroglia cell" or "glial cell" can be used interchangeably, referring to another large group of cells in the nerve tissue except for neurons, which are widely distributed in the central and peripheral nervous system. In mammals, the proportion of glial cells to neurons is about 10:1. The glial cells in the central nervous system mainly include astrocytes, NG2 glia, oligodendrocytes and microglia. Glial cells perform many physiological functions, including biochemical support (such as forming blood-brain barrier), provide nutrition for neurons, and maintain extracellular ion balance. In the state of injury or disease, glial cells will be activated and proliferated, and participate in the repair and scar formation after brain and spinal cord injury, but cannot differentiate into neurons. The key feature different from neural stem cells is that neural stem cells are self-replicating cells that have not fully differentiated, and have the potential to differentiate into neurons and various glial cells, while glial cells are end-differentiated cells.

The glial cells described in the invention are any astrocytes, NG2 glia, oligodendrocytes, microglia, or glial cells in the injured state, tumor cells derived from glial cells, etc. from human or non-human mammals; The glial cells in the injured state are glial cells in the state that the tissue or the surrounding environment of glial cells is in the state of mechanical trauma, stroke or neurodegenerative disease causing neuron death and apoptosis, which leads to the blockage or disorder of nerve signal transmission; The tumor cells derived from the glial cells are generally glioma cells, which are selected from astrocytoma, oligodendroglioma, ependymoma, mixed glioma, choroid plexus tumor, neuroepithelial histiocoma of uncertain origin, mixed tumor of neurons and neuroglia, pineal parenchyma tumor, embryonal tumor and neuroblastoma tumor derived from human or non-human mammals.

GLIOMA CELLS

As used in this article, the term "neuroglioma" is referred to as "glioma" for short, also known as " oligodendroglioma". It refers to all tumors of neuroepithelial origin in the broad sense, and tumors of various types of glial cells in the narrow sense. Glioma is one of the most lethal malignant tumors and the most common primary central nervous system tumor, accounting for 30% of brain and central nervous system tumors and 80% of brain malignant brain tumors. It is a serious threat to human health. According to the classification scheme of the World Health Organization (WHO) in 1999, it can be divided into astrocytoma, oligodendroglioma, ependymoma, mixed glioma, choroid plexus tumor, neuroepithelioma of uncertain origin, mixed neuroglioma and neuroglia, pineal parenchyma tumor, embryonal tumor and neuroblastoma tumor.

The glioma cells that can be used in the present invention are not particularly limited, including various gliomas from the mammalian central nervous system, such as astrocytoma, oligodendroglioma, ependymoma or neuroblastoma, preferably astrocytoma or neuroblastoma.

In the present invention, the transcription factor with trans-differentiation function and the combination of transcription factors have the ability to induce glioma cells to transform into neurons/neuron-like cells, and display the unique markers of neurons: DCX, Tuj 1, Map2, NeuN, Synapsin I. At the same time, the proliferation of glioma was significantly reduced, the tumor growth was slowed down, and the degree of malignancy was decreased.

DELIVERY SYSTEM

As used herein, the term "delivery system" has no special restriction. It can be an expression vector containing polynucleotide sequences encoding the transcription factors into glial cells or glioma cells. For example, virus vector can be any virus that can be used, and has the characteristics of transmitting its genome, bringing genetic material into other cells for infection. It can occur in intact living body or cell culture. Including lentivirus vector, adenovirus vector, adeno-associated virus vector, herpes virus vector, pox virus vector, etc.

The delivery system can also be a new type of nanoparticles, such as liposome nanoparticles, metal nanoparticles, polymer nanoparticles, etc., which are used to load the functional fragments of the transcription factor or the molecular entities that promote the expression or activity of the transcription factor, and deliver to the periphery of the target cell or enter the target cell.

The delivery system can also be an exocrine body that contains a functional segment of the transcription factor or a molecular entity that promotes the expression or activity of the transcription factor, or a modified red blood cell or bacteria that contains a functional segment of the transcription factor or a molecular entity that promotes the expression or activity of the transcription factor.

In addition, the delivery system can also combine molecules with targeted functions, such as specific monoclonal antibodies and polypeptides targeting glial cells or glioma cells, which can better promote the functional fragments of the transcription factor or promote the targeting of the functional fragments of the molecular entities with increased expression or activity of the transcription factor on glial cells or glioma cells, and increase the efficiency of inducing glial cell trans-differentiation and anti-tumor.

INDUCING METHOD

The invention also provides a method for inducing glial cells or glioma cells to differentiate into neuron cells or neuron-like cells in vitro and in vivo, so as to achieve the purpose of nerve repair or anti-tumor. The term "inducer" refers to any molecular entity that promotes the expression or activity enhancement of the transcription factor functional fragment of the present invention.

In vitro, the functional fragments containing the transcription factor or the molecular entity promoting the expression or activity enhancement of the functional fragments of the transcription factor and its delivery system can be contacted or applied (for example, injected) to the target cells cultured in vitro, so that the glial cells can be passively absorbed or reach the glial cells through the delivery system for effect, so as to achieve the differentiation of neurons in vitro and inhibit the proliferation of tumor cells. The cells successfully transdifferentiated in vitro can also be transplanted to achieve nerve repair at the nerve injury site.

In vivo, the functional fragments containing the transcription factor or the molecular entity promoting the expression or activity enhancement of the functional fragments of the transcription factor and its delivery system can be contacted or applied (for example, injected) to the nerve injury site or tumor focus, so that the glial cells can be passively absorbed or reach the glial cells through the delivery system for effect, so as to achieve the differentiation of neurons in vitro and inhibit the proliferation of tumor cells. The method of direct induction in vivo will help to repair nerve injury in situ and inhibit tumor in situ.

At the same time, in combination with molecular targeting technology, the delivery system containing the functional fragment of the transcription factor or the molecular entity that promotes the expression and activity of the functional fragment of the transcription factor is installed with a specific molecular target of glioma or glioma, which can achieve the induction of neuronal trans-differentiation through ectopic injection.

PHARMACEUTICAL COMPOSITION AND MODE OF ADMINISCTRATION

The invention also provides a drug composition, which contains any molecular entity or its delivery system that promotes the expression or activity enhancement of the transcription factor functional fragments, or the functional neuron group after the trans-differentiation of the transcription factor functional fragments or the molecular entity that promotes the expression and activity enhancement of the transcription factor functional fragments in vitro, as well as other pharmaceutically acceptable vectors.

The pharmaceutical composition of the invention usually contains AAV virus particles of $10^{10}$-$10^{13}$ PFU, preferably, AAV virus particles of $10^{11}$-$10^{13}$ PFU, and more preferably, AAV virus particles of $10^{10}$-$10^{12}$ PFU.

The pharmaceutical composition of the invention usually contains lentivirus particles of $10^7$-$10^{10}$ PFU, preferably, lentivirus particles of $10^7$-$10^9$ PFU, and more preferably, lentivirus particles of $10^8$-$10^9$ PFU.

The pharmaceutical composition of the invention usually contains adenovirus particles of $10^8$-$10^{11}$ PFU, preferably, adenovirus particles of $10^8$-$10^{10}$ PFU, and more preferably, adenovirus particles of $10^9$-$10^{10}$ PFU.

As used herein, the term "pharmaceutically acceptable carrier" refers to the carrier used for the administration of therapeutic agents, including various excipients and diluents. They are not necessary active ingredients themselves, and there is no excessive toxicity after application. A suitable carrier is well known to those skilled in the art. In the composition, the pharmaceutically acceptable carrier may contain liquid, such as water, saline and buffer. In addition, there may also be auxiliary substances in these carriers, such as fillers, lubricants, flow aids, wetting agents or emulsifiers, pH buffer substances, etc. The carrier can also contain cell transfection reagents.

Generally, the drug composition of the present invention can be obtained by mixing the expression vector with a pharmaceutically acceptable vector.

The method of administration of the composition described in the present invention is not particularly limited. Representative examples include but are not limited to: intravenous injection, subcutaneous injection, brain injection, intrathecal injection, spinal injection, etc.

THERAPEUTIC APPLICATION

The molecular entity or its delivery system containing any functional fragment of the transcription factor that promotes the expression or activity enhancement, or the functional nerve group described in the present invention can be used to prepare drugs for repairing nerve injury or inhibiting the proliferation and deterioration of glioma.

Beneficial effects:

The invention innovatively obtains a batch of transcription factors with reprogramming function, and explores the ability of transcription factors and their combinations to transdifferentiate, which can be potentially applied in different scenarios: for example, for the repair of nerve injury, the medium and high efficiency transcription factors and combinations of transcription factors can be selectively used according to the injury situation; For gliomas, a combination of transcription factors and transcription factors with higher transformation efficiency is needed to quickly regulate the malignant degree of gliomas.

The invention also further modifies the expression element of the transcription factor used for gene therapy, and significantly improves the efficiency of the transcription factor to promote glial cells to differentiate into neurons. In particular, the combination of transcription factors used in the present invention can transform human glioma cells into neurons, and cause glioma cells to withdraw from the cell cycle, thus no longer proliferate and grow. In the glioma model, the injection of adeno-associated virus containing the transcription factor combination can significantly reduce the tumor size and prolong the survival time of the animal.

Compared with the prior art, the main advantages of the invention include:

(1) No potential risk of tumorigenicity and immunogenicity;

(2) Without the influence of blood-brain barrier, glial cells undergo transformation through passive absorption or delivery system;

(3) It is more efficient than the existing transcription factors or combination of transcription factors, and has the potential of clinical application.

The present invention will be further described in combination with specific embodiments. It should be understood that these embodiments are only used to illustrate the invention and not to limit the scope of the invention. The following experimental methods without specific conditions are usually in accordance with conventional conditions, such as those described in the Molecular Cloning: Laboratory Manual (Sambrook et al., New York: Cold Spring Harbor Laboratory Press, 1989), or the conditions recommended by the manufacturer. Unless otherwise stated, percentages and portions are percentages and portions by weight.

Materials and methods

amino acid sequence

SEQ ID NO: 1(hNeuroD1 amino acid sequence)

MTKSYSESGLMGEPQPQGPPSWTDECLSSQDEEHEADKKEDDLETMNA
EEDSLRNGGEEEDEDEDLEEEEEEEEEDDDQKPKRRGPKKKKMTKARLERFK
LRRMKANARERNRMHGLNAALDNLRKVVPCYSKTQKLSKIETLRLAKNYIW
ALSEILRSGKSPDLVSFVQTLCKGLSQPTTNLVAGCLQLNPRTFLPEQNQDMPP
HLPTASASFPVHPYSYQSPGLPSPPYGTMDSSHVFHVKPPPHAYSAALEPFFES
PLTDCTSPSFDGPLSPPLSINGNFSFKHEPSAEFEKNYAFTMHYPAATLAGAQS
HGSIFSGTAAPRCEIPIDNIMSFDSHSHHERVMSAQLNAIFHD

SEQ ID NO: 2(mNeuroD1 amino acid sequence)

MTKSYSESGLMGEPQPQGPPSWTDECLSSQDEEHEADKKEDELEAMNA
EEDSLRNGGEEEEDEDLEEEEEEEEEEEDQKPKRRGPKKKKMTKARLERFK
LRRMKANARERNRMHGLNAALDNLRKVVPCYSKTQKLSKIETLRLAKNYIW
ALSEILRSGKSPDLVSFVQTLCKGLSQPTTNLVAGCLQLNPRTFLPEQNPDMPP

HLPTASASFPVHPYSYQSPGLPSPPYGTMDSSHVFHVKPPPHAYSAALEPFFES
PLTDCTSPSFDGPLSPPLSINGNFSFKHEPSAEFEKNYAFTMHYPAATLAGPQSH
GSIFSSGAAAPRCEIPIDNIMSFDSHSHHERVMSAQLNAIFHD

SEQ ID NO: 3(hNeuroD1 nucleotide sequence)

atgaccaaatcgtacagcgagagtgggctgatgggcgagcctcagccccaaggtcctccaagctggacagacga
gtgtctcagttctcaggacgaggagcacgaggcagacaagaaggaggacgacctcgaaaccatgaacgcagaggagg
actcactgaggaacggggggagaggaggaggacgaagatgaggacctggaagaggaggaagaagaggaagaggag
gatgacgatcaaaagcccaagagacgcggccccaaaaagaagaagatgactaaggctcgcctggagcgttttaaattga
gacgcatgaaggctaacgcccgggagcggaaccgcatgcacggactgaacgcggcgctagacaacctgcgcaaggtg
gtgccttgctattctaagacgcagaagctgtccaaaatcgagactctgcgcttggccaagaactacatctgggctctgtcgg
agatcctgcgctcaggcaaaagcccagacctggtctccttcgttcagacgctttgcaagggcttatcccaacccaccacca
acctggttgcgggctgcctgcaactcaatcctcggactttctgcctgagcagaaccaggacatgcccccccacctgccga
cggccagcgcttccttccctgtacacccctactcctaccagtcgcctgggctgcccagtccgccttacggtaccatggacag
ctcccatgtcttccacgttaagcctccgccgcacgcctacagcgcagcgctggagcccttctttgaaagccctctgactgatt
gcaccagcccttcctttgatggacccctcagcccgccgctcagcatcaatggcaacttctctttcaaacacgaaccgtccgc
cgagtttgagaaaaaattatgcctttaccatgcactatcctgcagcgacactggcaggggcccaaagccacggatcaatcttc
tcaggcaccgctgcccctcgctgcgagatccccatagacaatattatgtccttcgatagccattcacatcatgagcgagtcat
gagtgcccagctcaatgccatatttcatgattag

SEQIDNO: 4(mNeuroD1 nucleotide sequence)

atgaccaaatcatacagcgagagcgggctgatgggcgagcctcagccccaaggtcccccaagctggacagatga
gtgtctcagttctcaggacgaggaacacgaggcagacaagaaagaggacgagcttgaagccatgaatgcagaggagga
ctctctgagaaacggggggagaggaggaggaggaagatgaggatctagaggaagaggaggaagaagaagaggagga
ggaggatcaaaagcccaagagacggggtcccaaaaagaaaaagatgaccaaggcgcgcctagaacgttttaaattaag
gcgcatgaaggccaacgcccgcgagcggaaccgcatgcacgggctgaacgcggcgctggacaacctgcgcaaggtg
gtaccttgctactccaagacccagaaactgtctaaaatagagacactgcgcttggccaagaactacatctgggctctgtcag
agatcctgcgctcaggcaaaagccctgatctggtctccttcgtacagacgctctgcaaaggtttgtcccagcccactaccaa
tttggtcgccggctgcctgcagctcaaccctcggactttcttgcctgagcagaacccggacatgcccccgcatctgccaac
cgccagcgcttccttcccggtgcatccctactcctaccagtcccctggactgcccagcccgccctacggcaccatggacag
ctcccacgtcttccacgtcaagccgccgccacacgcctacagcgcagctctggagcccttctttgaaagcccccctaactga
ctgcaccagcccttcctttgacggacccctcagcccgccgctcagcatcaatggcaacttctctttcaaacacgaaccatcc
gccgagtttgaaaaaaattatgcctttaccatgcactaccctgcagcgacgctggcagggccccaaagccacggatcaatc
ttctcttccggtgccgctgcccctcgctgcgagatccccatagacaacattatgtctttcgatagccattcgcatcatgagcga
gtcatgagtgcccagcttaatgccatctttcacgattag

SEQIDNO: 5(hBrn2 amino acid sequence)

MATAASNHYSLLTSSASIVHAEPPGGMQQGAGGYREAQSLVQGDYGAL

QSNGHPLSHAHQWITALSHGGGGGGGGGGGGGGGGGGGGGGDGSPWSTSPL
GQPDIKPSVVVQQGGRGDELHGPGALQQQHQQQQQQQQQQQQQQQQQQQ
QQRPPHLVHHAANHHPGPGAWRSAAAAAHLPPSMGASNGGLLYSQPSFTVN
GMLGAGGQPAGLHHHGLRDAHDEPHHADHHPHPHSHPHQQPPPPPPPQGPP
GHPGAHHDPHSDEDTPTSDDLEQFAKQFKQRRIKLGFTQADVGLALGTLYGN
VFSQTTICRFEALQLSFKNMCKLKPLLNKWLEEADSSSGSPTSIDKIAAQGRK
RKKRTSIEVSVKGALESHFLKCPKPSAQEITSLADSLQLEKEVVRVWFCNRRQ
KEKRMTPPGGTLPGAEDVYGGSRDTPPHHGVQTPVQ

SEQIDNO: 6(mBrn2 amino acid sequence)

MATAASNHYSLLTSSASIVHAEPPGGMQQGAGGYREAQSLVQGDYGAL
QSNGHPLSHAHQWITALSHGGGGGGGGGGGGGGGGGGGGGGDGSPWSTSPL
GQPDIKPSVVVQQGGRGDELHGPGALQQQHQQQQQQQQQQQQQQQQQQQ
QQQQRPPHLVHHAANHHPGPGAWRSAAAAAHLPPSMGASNGGLLYSQPSFT
VNGMLGAGGQPAGLHHHGLRDAHDEPHHADHHPHPHSHPHQQPPPPPPPQG
PPGHPGAHHDPHSDEDTPTSDDLEQFAKQFKQRRIKLGFTQADVGLALGTLY
GNVFSQTTICRFEALQLSFKNMCKLKPLLNKWLEEADSSSGSPTSIDKIAAQG
RKRKKRTSIEVSVKGALESHFLKCPKPSAQEITSLADSLQLEKEVVRVWFCNR
RQKEKRMTPPGGTLPGAEDVYGGSRDTPPHHGVQTPVQ

SEQIDNO: 7(hBrn2 nucleotide sequence)

atggcgaccgcagcgtctaaccactacagcctgctcacctccagcgcctccatcgtgcacgccgagccgcccgg

cggcatgcagcagggcgcggggggctaccgcgaagcgcagagcctggtgcagggcgactacggcgctctgcagagc

aacggacacccgctcagccacgctcaccagtggatcaccgcgctgtcccacggcggcggcggcggggggcggtggcg

gcggcggggggggcggggcggcggcggggggcggcggcgacggctccccgtggtccaccagcccccctgggccag

ccggacatcaagccctcggtggtggtgcagcagggcggccgcggagacgagctgcacgggccaggcgccctgcagc

agcagcatcagcagcagcaacagcaacagcagcagcaacagcagcaacagcagcagcagcagcagcaacagcggc

cgccgcatctggtgcaccacgccgctaaccaccacccgggacccggggcatggcggagcgcggcggctgcagcgca

cctcccaccctccatgggagcgtccaacggcggcttgctctactcgcagcccagcttcacggtgaacggcatgctgggcg

ccggcgggcagccggccggtctgcaccaccacggcctgcgggacgcgcacgacgagccacaccatgccgaccacca

cccgcacccgcactcgcacccacaccagcagccgccgcccccgccgcccccgcagggtccgcctggccacccaggc

gcgcaccacgacccgcactcggacgaggacacgccgacctcggacgacctggagcagttcgccaagcagttcaagca

gcggcggatcaaactgggatttacccaagcggacgtggggctggctctgggcaccctgtatggcaacgtgttctcgcaga

ccaccatctgcaggtttgaggccctgcagctgagcttcaagaacatgtgcaagctgaagcctttgttgaacaagtggttgga

ggaggcggactcgtcctcgggcagccccacgagcatagacaagatcgcagcgcaagggcgcaagcggaaaaagcgg

acctccatcgaggtgagcgtcaagggggctctggagagccatttcctcaaatgccccaagccctcggcccaggagatcac

ctccctcgcggacagcttacagctggagaaggaggtggtgagagtttggttttgtaacaggagacagaaagagaaagga

tgaccctcccggagggactctgccgggcgccgaggatgtgtacgggggggagtagggacactccaccacaccacggg

gtgcagacgcccgtccagtga

SEQIDNO: 8(mBrn2 nucleotide sequence)

atggcgaccgcagcgtctaaccactacagcctgctcacctccagcgcctccatcgtacatgccgagccgcctggc ggcatgcagcagggcgcaggggggctaccgcgaggcgcagagcctggtgcagggcgactacggcgcgctgcagagc aacgggcacccgctcagccacgctcaccagtggatcaccgcgctgtcccacggcggcggcggcggggggcggcggcg gcggtggaggaggcggggggaggcggcggggggaggcggcgacggctccccgtggtccaccagcccccctaggccagc cggacatcaagccctcggtggtggtacagcagggtggccgaggcgacgagctgcacgggccaggagcgctgcagcaa cagcatcaacagcaacagcaacagcagcagcagcagcagcagcagcagcaacagcagcagcaacaacagc gaccgccacatctggtgcaccacgctgccaaccaccatcccgggcccggggcatggcggagtgcggcggctgcagctc acctccctccctccatgggagcttccaacggcggtttgctctattcgcagccgagcttcacggtgaacggcatgctgggcg caggagggcagccggctgggctgcaccaccacggcctgagggacgcccacgatgagccacaccatgcagaccacca cccgcatccgcactctcacccacaccagcaaccgcccccgccacctcccccacaaggcccaccgggccacccaggcg cgcaccacgacccgcactcggacgaggacacgccgacctcagacgacctggagcagttcgccaagcaattcaagcaga ggcggatcaaactcggatttactcaagcagacgtggggctggcgcttggcaccctgtacggcaacgtgttctcgcagacc accatctgcaggtttgaggccctgcagctgagcttcaagaacatgtgcaagctgaagcctttgttgaacaagtggttggaag aggcagactcatcctcgggcagccccaccagcatagacaagatcgcagcgcaagggcgcaaacggaaaaagcggacc tccatcgaggtgagcgtcaaggggcgctctggagagccatttcctcaaatgccctaagccctcggcccaggagatcacctc cctcgcggacagcttacagctggagaaggaggtggtgagagtttggttttgtaacaggagacagaaagagaaaaggatg acccctcccggagggactctgccgggcgccgaggatgtgtatgggggtagtagggacacgccaccacaccacggggt gcagacgcccgtccagtga

SEQIDNO: 9(hAscl1 amino acid sequence)

MESSAKMESGGAGQQPQPQPQQPFLPPAACFFATAAAAAAAAAAAAAQ SAQQQQQQQQQQQQQAPQLRPAADGQPSGGGHKSAPKQVKRQRSSSPELMRC KRRLNFSGFGYSLPQQQPAAVARRNERERNRVKLVNLGFATLREHVPNGAAN KKMSKVETLRSAVEYIRALQQLLDEHDAVSAAFQAGVLSPTISPNYSNDLNSM AGSPVSSYSSDEGSYDPLSPEEQELLDFTNWF

SEQIDNO: 10(mAscl1 amino acid sequence)

MESSGKMESGAGQQPQPPQPFLPPAACFFATAAAAAAAAAAAQSAQQ QQPQAPPQQAPQLSPVADSQPSGGGHKSAAKQVKRQRSSSPELMRCKRRLNF SGFGYSLPQQQPAAVARRNERERNRVKLVNLGFATLREHVPNGAANKKMSKV ETLRSAVEYIRALQQLLDEHDAVSAAFQAGVLSPTISPNYSNDLNSMAGSPVS SYSSDEGSYDPLSPEEQELLDFTNWF

SEQIDNO: 11(hAscl1 nucleotide sequence)

atggaaagctctgccaagatggagagcggcggcgccggccagcagccccagccgcagccccagcagcccttc

ctgccgcccgcagcctgtttctttgccacggccgcagccgcggcggccgcagccgccgcagcggcagcgcagagcgc
gcagcagcagcagcagcagcagcagcagcagcagcaggcgccgcagctgagaccggcggccgacggccagccctc
aggggggcggtcacaagtcagcgcccaagcaagtcaagcgacagcgctcgtcttcgcccgaactgatgcgctgcaaacg
ccggctcaacttcagcggctttggctacagcctgccgcagcagcagccggccgccgtggcgcgccgcaacgagcgcga
gcgcaaccgcgtcaagttggtcaacctgggctttgccacccttcgggagcacgtccccaacggcgcggccaacaagaag
atgagtaaggtggagacactgcgctcggcggtcgagtacatccgcgcgctgcagcagctgctggacgagcatgacgcg
gtgagcgccgccttccaggcaggcgtcctgtcgcccaccatctcccccaactactccaacgacttgaactccatggccgg
ctcgccggtctcatcctactcgtcggacgagggctcttacgacccgctcagccccgaggagcaggagcttctcgacttcac
caactggttctga

SEQIDNO: 12(mAscl1 nucleotide sequence)

atggagagctctggcaagatggagagtggagccggccagcagccgcagcccccgcagcccttcctgcctcccg
cagcctgcttctttgcgaccgcggcggcggcggcagcggcggcggccgcggcagctcagagcgcgcagcagcaaca
gccgcaggcgccgccgcagcaggcgccgcagctgagcccggtggccgacagccagccctcaggggggcggtcacaa
gtcagcggccaagcaggtcaagcgccagcgctcgtcctctccggaactgatgcgctgcaaacgccggctcaacttcagc
ggcttcggctacagcctgccacagcagcagccggccgccgtggcgcgccgcaacgagcgcgagcgcaaccgggtca
agttggtcaacctgggtttttgccaccctccgggagcatgtccccaacggcgcggccaacaagaagatgagcaaggtgga
gacgctgcgctcggcggtcgagtacatccgcgcgctgcagcagctgctggacgagcacgacgcggtgagcgctgccttt
caggcgggcgtcctgtcgcccaccatctcccccaactactccaacgacttgaactctatggcgcggttctccggtctcgtcct
actcctccgacgagggatcctacgaccctcttagcccagaggaacaagagctgctggactttaccaactggttctga

SEQIDNO: 13(hNgn2 amino acid sequence)

MFVKSETLELKEEEDVLVLLGSASPALAALTPLSSSADEEEEEEPGASGGA
RRQRGAEAGQGARGGVAAGAEGCRPARLLGLVHDCKRRPSRARAVSRGAKT
AETVQRIKKTRRLKANNRERNRMHNLNAALDALREVLPTFPEDAKLTKIETL
RFAHNYIWALTETLRLADHCGGGGGGLPGALFSEAVLLSPGGASAALSSSGDS
PSPASTWSCTNSPAPSSSVSSNSTSPYSCTLSPASPAGSDMDYWQPPPPDKHRY
APHLPIARDCI

SEQIDNO: 14(mNgn2 amino acid sequence)

MFVKSETLELKEEEVLMLLGSASPASATLTPMSSSADEEEDEELRRPGSA
RGQRGAEAGQGVQGSPASGAGGCRPGRLLGLMHECKRRPSRSRAVSRGAKT
AETVQRIKKTRRLKANNRERNRMHNLNAALDALREVLPTFPEDAKLTKIETL
RFAHNYIWALTETLRLADHCAGAGGLQGALFTEAVLLSPGAALGASGDSPSPP
SSWSCTNSPASSSNSTSPYSCTLSPASPGSDVDYWQPPPPEKHRYAPHLPLARD
CI

SEQIDNO: 15(hNgn2 nucleotide sequence)

atgttcgtcaaatccgagaccttggagttgaaggaggaagaggacgtgttagtgctgctcggatcggcctcccccg ccttggcggccctgaccccgctgtcatccagcgccgacgaagaagaggaggaggagccgggcgcgtcaggcggggc gcgtcggcagcgcggggctgaggccgggcaggggggcgcggggcggcgtggctgcgggtgcggagggctgccggc ccgcacggctgctgggtctggtacacgattgcaaacggcgcccttcccgggcgcgggccgtctcccgaggcgccaaga cggccgagacggtgcagcgcatcaagaagacccgtagactgaaggccaacaaccgcgagcgaaaccgcatgcacaac ctcaacgcggcactggacgcgctgcgcgaggtgctccccacgttccccgaggacgccaagctcaccaagatcgagacc ctgcgcttcgcccacaactacatctgggcactcaccgagaccctgcgcctggcggatcactgcggggggcggcggcggg ggcctgccggggggcgctcttctccgaggcagtgttgctgagcccgggaggagccagcgccgccctgagcagcagcgg agacagcccctcgcccgcctccacgtggagttgcaccaacagccccgcgccgtcctcctccgtgtcctccaattccacctc cccctacagctgcactttatcgcccgccagcccggccgggtcagacatggactattggcagcccccacctcccgacaagc accgctatgcacctcacctccccatagccagggattgtatctag

SEQIDNO: 16(mNgn2 nucleotide sequence)

atgttcgtcaaatctgagactctggagttgaaggaggaagaggaggtactgatgctgctgggctcggcttccccgg cctcggcgaccctgaccccgatgtcctccagcgcggacgaggaggaggacgaggagctgcgccggccgggctccgc gcgtgggcagcgtggagcggaagccgggcaggggggtgcagggcagtccggcgtcgggtgccggggggttgccggcc agggcggctgctgggcctgatgcacgagtgcaagcgtcgcccgtcgcgctcacgggccgtctcccgaggtgccaagac ggcggagacggtgcagcgcatcaagaagacccgcaggctcaaggccaacaaccgcgagcgcaaccgcatgcacaac ctaaacgccgcgctggacgcgctgcgcgaggtgctgcccaccttccccgaggatgccaagctcacgaagatcgagacg ctgcgcttcgcccacaattacatctgggcgctcaccgagactctgcgcctggcggaccactgcgccggcgccggtggcct ccaggggggcgctcttcacggaggcggtgctcctgagcccgggagctgcgctcggcgccagcggggacagcccttctcc accttcctcctggagctgcaccaacagccccggcgtcatcctccaactccacgtccccatacagctgcactttatcgcccgct agccccgggtcagacgtggactactggcagcccccacctccggagaagcatcgttatgcgcctcacctgcccctcgcca gggactgtatctag

SEQIDNO: 17(hGsx1 amino acid sequence)

MPRSFLVDSLVLREAGEKKAPEGSPPPLFPYAVPPPHALHGLSPGACHAR
KAGLLCVCPLCVTASQLHGPPGPPALPLLKASFPPFGSQYCHAPLGRQHSAVSP
GVAHGPAAAAAAAALYQTSYPLPDPRQFHCISVDSSSNQLPSSKRMRTAFTST
QLLELEREFASNMYLSRLRRIEIATYLNLSEKQVKIWFQNRRVKHKKEGKGSN
HRGGGGGGAGGGGSAPQGCKCASLSSAKCSEDDDELPMSPSSSGKDDRDLT
VTP

SEQIDNO: 18(mGsx1 amino acid sequence)

MPRSFLVDSLVLREASDKKAPEGSPPPLFPYAVPPPHALHGLSPGACHAR
KAGLLCVCPLCVTASQLHGPPGPPALPLLKASFPPFGSQYCHAPLGRQHSVSP
GVAHGPAAAAAAAALYQTSYPLPDPRQFHCISVDSSSNQLPSSKRMRTAFTST
QLLELEREFASNMYLSRLRRIEIATYLNLSEKQVKIWFQNRRVKHKKEGKGSN

HRGGAGAGAGGGAPQGCKCSSLSSAKCSEDDDELPMSPSSSGKDDRDLTVTP

SEQIDNO: 19(hGsx1 nucleotide sequence)

atgccgcgctccttcctggtggactcgctagtgctgcgcgaggcgggcgagaagaaggcgcccgagggcagcc
cgccgccgctcttcccctacgctgtgccccgccgcacgcgctgcacggtctctcgcctggcgcctgccacgcgcgcaa
ggctgggctgctgtgcgtgtgcccgctctgcgtcaccgcctcgcagctgcatgggccccccgggccgcccgcgctgcct
ctactcaaggcttccttcccacccttcggctcgcagtactgccacgcgcccctgggccgccagcactctgctgtgtcgccc
ggggtcgctcacggcccggccgccgctgctgctgccgccgcgctctaccagacctcctacccgctgcctgaccccaggc
agttccactgcatctctgtggacagcagctctaaccagctgcccagcagcaagaggatgcgcacggctttcaccagcacg
cagctgctagagctggagcgcgagttcgcttctaatatgtacctgtcccgcctacgtcgcatcgagatcgcgacctacctga
atctgtccgagaagcaggtgaagatctggtttcagaaccgccgagtgaagcacaagaaggagggcaagggcagcaacc
atcgtggcggcggcggcgggggtgccggtggtggcgggagcgcaccgcaaggctgcaagtgcgcatcgctctcctca
gccaagtgctccgaggatgacgacgaattgcccatgtctccgtcctcctcagggaaggacgaccgggatcttacggtcact
ccctag

SEQIDNO: 20(mGsx1 nucleotide sequence)

atgccgcgctccttcctggtggattcccttgtgctgcgggaagccagcgacaagaaggctccggagggcagcccg
ccaccgctcttcccctacgcggtcccgccgccgcacgcgctccacggcctctcgccgggcgcctgccacgcgcgcaag
gccggcttgctgtgcgtgtgtccctctgtgtcaccgcttcgcagctgcacgggccccccgggccgccggcactgccgct
actcaaggcgtccttccctcccttcggatcgcagtactgccacgcaccccctgggccgccagcactccgtgtcccctggagt
cgcccacggcccggctgcggccgcagcagctgctgcactctaccagacctcctacccgctgccggatcccagacagttt
cactgcatctctgtggacagcagctcgaaccagctgcccagcagcaagaggatgcggacggcgttcaccagcacacag
ctcctggagctggagcgagagttcgcctccaacatgtacctctcccgcctgcggcgcatcgagatcgcgacctatctgaac
ctgtccgagaagcaggtgaagatctggtttcagaaccgccgggtgaagcacaagaaagaaggcaaaggcagtaaccac
cgcggcggagctggggcggggggccggcggggggcgcaccgcaaggctgcaagtgctcttcgctctcctcagccaaatg
ctcagaggacgacgacgaattgcccatgtctccatcttcctccgggaaggatgacagagatctcacagtcactccgtag

SEQIDNO: 21(hTbr1 amino acid sequence)

MQLEHCLSPSIMLSKKFLNVSSSYPHSGGSELVLHDHPIISTTDNLERSSPL
KKITRGMTNQSDTDNFPDSKDSPGDVQRSKLSPVLDGVSELRHSFDGSAADR
YLLSQSSQPQSAATAPSAMFPYPGQHGPAHPAFSIGSPSRYMAHHPVITNGAYN
SLLSNSSPQGYPTAGYPYPQQYGHSYQGAPFYQFSSTQPGLVPGKAQVYLCN
RPLWLKFHRHQTEMIITKQGRRMFPFLSFNISGLDPTAHYNIFVDVILADPNH
WRFQGGKWVPCGKADTNVQGNRVYMHPDSPNTGAHWMRQEISFGKLKLTN
NKGASNNNGQMVVLQSLHKYQPRLHVVEVNEDGTEDTSQPGRVQTFTFPET
QFIAVTAYQNTDITQLKIDHNPFAKGFRDNYDTIYTGCDMDRLTPSPNDSPRSQ
IVPGARYAMAGSFLQDQFVSNYAKARFHPGAGAGPGPGTDRSVPHTNGLLSP
QQAEDPGAPSPQRWFVTPANNRLDFAASAYDTATDFAGNAATLLSYAAAGVK

ALPLQAAGCTGRPLGYYADPSGWGARSPPQYCGTKSGSVLPCWPNSAAAAA
RMAGANPYLGEEAEGLAAERSPLPPGAAEDAKPKDLSDSSWIETPSSIKSIDSS
DSGIYEQAKRRRISPADTPVSESSSPLKSEVLAQRDCEKNCAKDISGYYGFYSH
S

SEQIDNO: 22(mTbr1 amino acid sequence)

MQLEHCLSPSIMLSKKFLNVSSSYPHSGGSELVLHDHPIISTTDNLERSSPL
KKITRGMTNQSDTDNFPDSKDSPGDVQRSKLSPVLDGVSELRHSFDGSAADR
YLLSQSSQPQSAATAPSAMFPYPSQHGPAHPAFSIGSPSRYMAHHPVITNGAYN
SLLSNSSPQGYPTAGYPYPQQYGHSYQGAPFYQFSSTQPGLVPGKAQVYLCN
RPLWLKFHRHQTEMIITKQGRRMFPFLSFNISGLDPTAHYNIFVDVILADPNH
WRFQGGKWVPCGKADTNVQGNRVYMHPDSPNTGAHWMRQEISFGKLKLTN
NKGASNNNGQMVVLQSLHKYQPRLHVVEVNEDGTEDTSQPGRVQTFTFPET
QFIAVTAYQNTDITQLKIDHNPFAKGFRDNYDTIYTGCDMDRLTPSPNDSPRSQ
IVPGARYAMAGSFLQDQFVSNYAKARFHPGAGAGPGPGTDRSVPHTNGLLSP
QQAEDPGAPSPQRWFVTPANNRLDFAASAYDTATDFAGNAATLLSYAAAGVK
ALPLQAAGCTGRPLGYYADPSGWGARSPPQYCGAKSGSVLPCWPNSAAAAA
RMAGANPYLGEEAEGLAAERSPLAPAAEDAKPKDLSDSSWIETPSSIKSIDSSD
SGIYEQAKRRRISPADTPVSESSSPLKSEVLAQRDCEKNCAKDIGGYYGFYSHS

SEQIDNO: 23(hTbr1 nucleotide sequence)

atgcagctggagcactgcctttctccttctatcatgctctccaagaaatttctcaatgtgagcagcagctacccacattc
aggcggatccgagcttgtcttgcacgatcatcccattatctcgaccactgacaacctggagagaagttcacctttgaaaaaa
attaccagggggatgacgaatcagtcagatacagacaattttcctgactccaaggactcaccaggggacgtccagagaag
taaactctctcctgtcttggacgggggtctctgagcttcgtcacagtttcgatggctctgctgcagatcgctacctcctctctcagt
ccagccagccacagtctgcggccactgctcccagtgccatgttcccgtaccccggccagcacggaccggcgcaccccg
ccttctccatcggcagccctagccgctacatggcccaccacccggtcatcaccaacggagcctacaacagcctcctgtcca
actcctcgccgcagggataccccacggccggctacccctacccacagcagtacggccactcctaccaaggagctccgtt
ctaccagttctcctccacccagccgggggctggtgcccggcaaagcacaggtgtacctgtgcaacaggccccctttggctga
aatttcaccggcaccaaacggagatgatcatcaccaaacagggaaggcgcatgtttcctttttttaagttttaacatttctggtct
cgatcccacggctcattacaatattttgtggatgtgattttggcggatcccaatcactggaggtttcaaggaggcaaatgggt
tccttgcggcaaagcggacaccaatgtgcaaggaaatcgggtctatatgcatccggattcccccaacactggggctcactg
gatgcgccaagaaatctcttttggaaaattaaaacttacgaacaacaaaggagcttcaaataacaatgggcagatggtggttt
tacagtccttgcacaagtaccagccccgcctgcatgtggtggaagtgaacgaggacggcacggaggacactagccagcc
cggccgcgtgcagacgttcactttccctgagactcagttcatcgccgtcaccgcctaccagaacacggatattacacaactg
aaaatagatcacaaccccttttgcaaaaggatttcgggataattatgacacgatctacaccggctgtgacatggaccgcctga
ccccctcgcccaacgactcgccgcgctcgcagatcgtgcccggggcccgctacgccatggccggctctttcctgcagga
ccagttcgtgagcaactacgccaaggcccgcttccacccgggcgcgggcgcgggccccgggccgggtacggaccgca

gcgtgccgcacaccaacgggctgctgtcgccgcagcaggccgaggacccgggcgcgccctcgccgcaacgctggttt
gtgacgccggccaacaaccggctggacttcgcggcctcggcctatgacacggccacggacttcgcgggcaacgcggcc
acgctgctctcttacgcggcggcgggcgtgaaggcgctgccgctgcaggctgcaggctgcactggccgcccgctcggct
actacgccgacccgtcgggctggggcgcccgcagtcccccgcagtactgcggcaccaagtcgggctcggtgctgccct
gctggcccaacagcgccgcggccgccgcgcgcatggccggcgccaatccctacctgggcgaggaggccgagggcct
ggccgccgagcgctcgccgctgccgcccggcgccgccgaggacgccaagcccaaggacctgtccgattccagctgga
tcgagacgccctcctcgatcaagtccatcgactccagcgactcggggatttacgagcaggccaagcggaggcggatctc
gccggccgacacgcccgtgtccgagagttcgtccccgctcaagagcgaggtgctggcccagcgggactgcgagaaga
actgcgccaaggacattagcggctactatggcttctactcgcacagctag

SEQIDNO: 24(mTbr1 nucleotide sequence)

atgcagctggagcattgcctctctccttctatcatgctctccaagaaatttctcaatgtgagcagcagctacccacattc gggcggatctgagcttgtcttgcatgatcatcccattatctcgaccactgacaacctggagagaagttcacctttgaaaaaaa ttaccaggggatgacgaatcagtcagatacagacaattttcctgactccaaggactcaccaggggacgtccagagaagt aaactctctcctgtcttggacggggtctctgagcttcgtcacagtttcgatggctctgctgcagatcgttacctactctctcagtc cagccagccacagtctgcggccaccgctcccagtgccatgttcccgtaccccagccagcacggaccggcgcatcccgc cttctccatcggcagccccagtcgctacatggcccaccacccggtcattaccaacggagcttacaacagcctgctgtccaa ctcttcgccgcagggctaccccacggccggctacccctacccacagcagtacggccactcctaccaaggagccccttct accagttctcctccacccagcccgggttggtgcccggcaaggcgcaagtatacctgtgcaacaggccactttggctgaaat ttcatcggcatcaaacggagatgatcatcactaaacagggaaggcgcatgtttcccttttttgagttttaacatttctggtctcgat cccaccgctcattacaatattttgtggatgtgattttggcggatcccaatcactggaggtttcaaggaggcaaatggggttcctt gtggcaaagcggacaccaatgtgcaaggaaaccgggtctatatgcatccggattcccccaacactggggctcactggatg cggcaagaaatctcttttggaaaattaaaacttaccaacaacaagggagcatcaaacaacaatgggcagatggtggttttac agtccctgcacaagtaccagccccgtctgcacgtggtggaagtgaatgaggatggcacagaggacaccagccagccag gccgagtccagacgttcacttttccggagactcagttcatcgctgtcaccgcctaccagaacacggatattacacaactaaa aatagatcataaccccctttgcaaaaggattcgagataactatgacacgatctacacgggctgcgacatggaccgcttgacc ccgtcgcccaacgactctccgcgctcgcagatcgtgcccggcgcccgctacgccatggccggctctttcctgcaagacca gttcgtgagcaactacgccaaggcccgcttccacccggggcgccggcgcgggtcccgggccgggcacggaccgcagc gtgccgcacaccaacgggctgctgtccccgcagcaggccgaggacccgggcgcgccgtcgccgcagcgctggttcgt cacgccggccaacaaccggctggacttcgcggcctcggcctacgacacggcacggacttcgccggcaacgcggcca cgctgctgtcgtatgcggccgcgggcgtgaaggcgctgcccttgcaggccgcggggctgcacgggccgcccgctcggct actacgccgaccttcgggctggggcgcgcgcagcccccccgcagtactgcggcgccaagtcgggctccgtgctcccct gctggcccaacagcgccgcggccgccgcgcgcatggccggcgccaaccccctatctgggcgaggaggccgagggcct ggcggccgagcgctcgccgctggcgcccgccgccgaggacgccaagcccaaggacctgtccgactccagctggatcg agacgccctcctccatcaaatccatcgactccagcgactcggggatttacgagcaggccaagcggaggcggatctcgcc ggctgacacgccggtgtctgagagctcgtccccgctcaagagcgaggtgctggcccagcgggactgcgagaagaactg cgccaaggacataggcggctactatggcttctactcgcacagctag

SEQIDNO: 25(hDlx2 amino acid sequence)

MTGVFDSLVADMHSTQIAASSTYHQHQQPPSGGGAGPGGNSSSSSSLHK
PQESPTLPVSTATDSSYYTNQQHPAGGGGGGGSPYAHMGSYQYQASGLNNVP
YSAKSSYDLGYTAAYTSYAPYGTSSSPANNEPEKEDLEPEIRIVNGKPKKVRKP
RTIYSSFQLAALQRRFQKTQYLALPERAELAASLGLTQTQVKIWFQNRRSKFK
KMWKSGEIPSEQHPGASASPPCASPPVSAPASWDFGVPQRMAGGGGPGSGGS
GAGSSGSSPSSAASAFLGNYPWYHQTSGSASHLQATAPLLHPTQTPQPHHHHH
HHGGGGAPVSAGTIF

SEQIDNO: 26(mDlx2 amino acid sequence)

MTGVFDSLVADMHSTQITASSTYHQHQQPPSGAGAGPGGNSNSSSSNSSL HKPQESPTLPVSTATDSSYYTNQQHPAGGGGGGGASPYAHMGSYQYHASGLNN VSYSAKSSYDLGYTAAYTSYAPYGTSSSPVNNEPDKEDLEPEIRIVNGKPKKV RKPRTIYSSFQLAALQRRFQKTQYLALPERAELAASLGLTQTQVKIWFQNRRS KFKKMWKSGEIPTEQHPGASASPPCASPPVSAPASWDFGAPQRMAGGGPGSG GGGAGSSGSSPSSAASAFLGNYPWYHQASGSASHLQATAPLLHPSQTPQAHH HHHHHHHAGGGAPVSAGTIF

SEQIDNO: 27(hDlx2 nucleotide sequence)

atgactggagtctttgacagtctagtggctgatatgcactcgacccagatcgccgcctccagcacgtaccaccagca
ccagcagcccccgagcggcggcggcgccggcccgggtggcaacagcagcagcagcagcagcctccacaagcccca
ggagtcgcccacccttccggtgtccaccgccaccgacagcagctactacaccaaccagcagcacccggcgggcggcg
gcggcggcggggggctcgccctacgcgcacatgggttcctaccagtaccaagccagcggcctcaacaacgtcccttactc
cgccaagagcagctatgacctgggctacaccgccgcctacacctcctacgctccctatggaaccagttcgtccccagcca
acaacgagcctgagaaggaggaccttgagcctgaaattcggatagtgaacgggaagccaaagaaagtccggaaacccc
gcaccatctactccagtttccagctggcggctcttcagcggcgtttccaaaagactcaatacttggccttgccggagcgagc
cgagctggcggcctctctgggcctcacccagactcaggtcaaaatctggttccagaaccgccggtccaagttcaagaaga
tgtggaaaagtggtgagatcccctcggagcagcaccctggggccagcgcttctccaccttgtgcttcgccgccagtctcag
cgccggcctcctgggactttggtgtgccgcagcggatggcgggcggcggtggtccgggcagtggcggcagcggcgcc
ggcagctcgggctccagcccgagcagcgcggcctcggctttctgggcaactacccctggtaccaccagacctcgggat
ccgcctcacacctgcaggccacggcgccgctgctgcacccactcagaccccgcagccgcatcaccaccaccaccatc
acggcggcggggggcgccccggtgagcgcggggacgattttctaa

SEQIDNO: 28(mDlx2 nucleotide sequence)

atgactggagtctttgacagtctggtggctgatatgcactcgacccagatcaccgcctccagcacgtaccaccagca
ccagcagcccccgagcggtgcgggcgccggccctggcggcaacagcaacagcagcagcagcaacagcagcctgca
caagccccaggagtcgccaaccctcccggtgtccacggctacggacagcagctactacaccaaccagcagcacccggc
gggcggcggcggcggggggggcctcgccctacgcgcacatgggctcctaccagtaccacgccagcggcctcaacaatg
tctcctactccgccaaaagcagctacgacctgggctacaccgccgcgtacacctcctacgcgccctacggcaccagttcgt
ctccggtcaacaacgagccggacaaggaagaccttgagcctgaaatccgaatagtgaacgggaagccaaagaaagtcc
ggaaaccacgcaccatctactccagtttccagctggcggcccttcaacgacgcttccagaagacccagtatctggccctgc
cagagcgagccgagctggcggcgtccctgggcctcacccaaactcaggtcaaaatctggttccagaaccgccgatccaa
gttcaagaagatgtggaaaagcggcgagatacccaccgagcagcaccctggagccagcgcttctcctccttgtgcctccc
cgccggtctcggcgccagcatcctgggacttcggcgcgccgcagcggatggctggcggcggcccgggcagcggagg
cggcggtgcgggcagctctggctccagcccgagcagcgccgcctcggcctttctgggaaactacccgtggtaccaccag
gcttcgggctccgcttcacacctgcaggccacagcgccacttctgcatccttcgcagactccgcaggcgcaccatcaccac
catcaccaccaccacgcaggcggggggcgccccggtgagcgcggggacgattttctaa

SEQIDNO: 29(hPtfla amino acid sequence)

MDAVLLEHFPGGLDAFPSSYFDEDDFFTDQSSRDPLEDGDELLADEQAE
VEFLSHQLHEYCYRDGACLLLQPAPPAAPLALAPPSSGGLGEPDDGGGGGYC
CETGAPPGGFPYSPGSPPSCLAYPCAGAAVLSPGARLRGLSGAAAAAARRRRR
VRSEAELQQLRQAANVRERRRMQSINDAFEGLRSHIPTLPYEKRLSKVDTLRL
AIGYINFLSELVQADLPLRGGGAGGCGGPGGGGRLGGDSPGSQAQKVIICHRG
TRSPSPSDPDYGLPPLAGHSLSWTDEKQLKEQNIIRTAKVWTPEDPRKLNSKSS
FNNIENEPPFEFVS

SEQIDNO: 30(mPtfla amino acid sequence)

MDAVLLEHFPGGLDTFPSPYFDEEDFFTDQSSRDPLEDSDELLGDEQAEV
EFLSHQLHEYCYRDGACLLLQPAPSAAPHALAPPPLGDPGEPEDNVSYCCDA
GAPLAAFPYSPGSPPSCLAYPCAAVLSPGARLGGLNGAAAAAARRRRRVRS
EAELQQLRQAANVRERRRMQSINDAFEGLRSHIPTLPYEKRLSKVDTLRLAIG
YINFLSELVQADLPLRGSGAGGCGGPGGSRHLGEDSPGNQAQKVIICHRGTRS
PSPSDPDYGLPPLAGHSLSWTDEKQLKEQNIIRTAKVWTPEDPRKLNSKSFDNI
ENEPPFEFVS

SEQIDNO: 31(hPtf1a nucleotide sequence)

atggacgcggtgttgctggagcacttccccggggggcctagacgcctttccttcttcgtacttcgacgaggacgacttc
ttcaccgaccagtcttcacgggacccccctggaggacggcgatgagctgctggcggacgagcaggccgaggtggagttc
cttagccaccagctccacgagtactgctaccgcgacgggggcgtgcctgctgctgcagcccgcgcccccggccgccccg
ctagcgctcgccccgccgtcctcggggggcctcggtgagccagacgacggcggcggcggcggctactgctgcgagac
ggggggcgccccaggcggcttcccctactcgcccggctcgccgccctcgtgcctggcctacccgtgcgccggggcggc
agtactgtctcccggggcgcggctgcgcggcctgagcggagcggcggctgcggcggcgcggcgccggcggcgggtg
cgctccgaggcggagctgcagcagctgcggcaggcggccaacgtgcgcgagcggcggcgcatgcagtccatcaacg
acgccttcgagggggctgcgctcgcacatccccacgctgccctacgagaagcgcctctccaaggtggacacgctgcgcct
ggccatcggctacatcaacttcctcagcgagctcgtgcaggccgacctgcccttgcgcggcggtggcgcgggcggctgc

ggggggccgggcggcggcgggcgcctgggcggggacagcccgggcagccaggcccagaaggtcatcatctgccat
cggggcacccggtccccctcccccagcgaccctgattatggcctccctcccctagcaggacactctctctcatggactgat
gaaaaacaactcaaggaacaaaatattatccgaacagccaaagtctggaccccagaggaccccagaaaactcaacagca
aatcttccttcaacaacatagaaacgaaccaccatttgagtttgtgtcctga

SEQIDNO: 32(mPtf1a nucleotide sequence)

atggacgccgtactcctggagcacttccccggggggcctggacaccttcccatccccttactttgatgaggaagatttc
ttcaccgaccagtcctctcgggacccgctggaggacagcgacgagctgctgggggacgagcaagcagaagtagagttc
ctcagccaccagctacacgaatactgctaccgcgacggggcgtgcctgctgctgcaacccgcgccctcggccgccccgc
acgcgctcgccccgccgcctttggggggatcctggcgagcccgaggacaacgtcagctattgctgcgatgcaggggctcc
tctcgctgccttcccctactcgcctggctcaccgccctcgtgcctcgcctacccgtgtgccgcggtgctgtcccccggtgcg
cggctcggtggtttgaacggggctgcggcagcggcggcagcaaggcggcggcgacgcgtgcgctccgaggcggagc
tgcagcagctgcgacaagccgctaatgtgcgagagcggcgccgcatgcagtccatcaacgacgccttcgaggggctgc
gttcgcacatccccacgctaccctacgaaaagcgcctctccaaagtagacacgctgcgcttggccataggctacattaactt
cctcagcgagctggtgcaagccgacctgccgctgcgcggggagtggcgcaggtggttgcggggggcccaggtggcagcc
ggcacctcggagaggacagtcccggtaaccaggcccagaaggttatcatctgccatcgaggcacccgttcaccctcccc
cagtgacccggattatggtctccctcctcttgcaggcactctctttcctggactgatgaaaaacagctcaaagaacaaaata
tcatccgtacagctaaagtgtggaccccagaggaccccagaaaactcaacagtaaatctttcgacaacatagagaacgaa
ccaccctttgagtttgtgtcctga

SEQIDNO: 33(hPax6 amino acid sequence)

MQNSHSGVNQLGGVFVNGRPLPDSTRQKIVELAHSGARPCDISRILQVSN
GCVSKILGRYYETGSIRPRAIGGSKPRVATPEVVSKIAQYKRECPSIFAWEIRDR
LLSEGVCTNDNIPSVSSINRVLRNLASEKQQMGADGMYDKLRMLNGQTGSW
GTRPGWYPGTSVPGQPTQDGCQQQEGGGENTNSISSNGEDSDEAQMRLQLK
RKLQRNRTSFTQEQIEALEKEFERTHYPDVFARERLAAKIDLPEARIQVWFSNR
RAKWRREEKLRNQRRQASNTPSHIPISSSFSTSVYQPIPQPTTPVSSFTSGSMLG
RTDTALTNTYSALPPMPSFTMANNLPMQPPVPSQTSSYSCMLPTSPSVNGRSY
DTYTPPHMQTHMNSQPMGTSGTTSTGLISPGVSVPVQVPGSEPDMSQYWPRL
Q

SEQIDNO: 34(mPax6 amino acid sequence)

MQNSHSGVNQLGGVFVNGRPLPDSTRQKIVELAHSGARPCDISRILQTHA
DAKVQVLDNENVSNGCVSKILGRYYETGSIRPRAIGGSKPRVATPEVVSKIAQ
YKRECPSIFAWEIRDRLLSEGVCTNDNIPSVSSINRVLRNLASEKQQMGADGM
YDKLRMLNGQTGSWGTRPGWYPGTSVPGQPTQDGCQQQEGGGENTNSISSN
GEDSDEAQMRLQLKRKLQRNRTSFTQEQIEALEKEFERTHYPDVFARERLAA
KIDLPEARIQVWFSNRRAKWRREEKLRNQRRQASNTPSHIPISSSFSTSVYQPIP

QPTTPVSSFTSGSMLGRTDTALTNTYSALPPMPSFTMANNLPMQPPVPSQTSS
YSCMLPTSPSVNGRSYDTYTPPHMQTHMNSQPMGTSGTTSTGLISPGVSVPV
QVPGSEPDMSQYWPRLQ

SEQIDNO: 35(hPax6 nucleotide sequence)

atgcagaacagtcacagcggagtgaatcagctcggtggtgtctttgtcaacgggcggccactgccggactccacc
cggcagaagattgtagagctagctcacagcggggcccggccgtgcgacatttcccgaattctgcaggtgtccaacggatg
tgtgagtaaaattctgggcaggtattacgagactggctccatcagacccagggcaatcggtggtagtaaaccgagagtagc
gactccagaagttgtaagcaaaatagcccagtataagcgggagtgcccgtccatctttgcttgggaaatccgagacagatta
ctgtccgaggggggtctgtaccaacgataacataccaagcgtgtcatcaataaacagagttcttcgcaacctggctagcgaa
aagcaacagatgggcgcagacggcatgtatgataaactaaggatgttgaacgggcagaccggaagctggggcacccgc
cctggttggtatccggggacttcggtgccagggcaacctacgcaagatggctgccagcaacaggaaggaggggggagag
aataccaactccatcagttccaacggagaagattcagatgaggctcaaatgcgacttcagctgaagcggaagctgcaaag
aaatagaacatcctttacccaagagcaaattgaggccctggagaaagagtttgagagaacccattatccagatgtgtttgcc
cgagaaagactagcagccaaaatagatctacctgaagcaagaatacaggtatggttttctaatcgaagggccaaatggaga
agagaagaaaaactgaggaatcagagaagacaggccagcaacacacctagtcatattcctatcagcagtagtttcagcac
cagtgtctaccaaccaattccacaacccaccacaccggtttcctccttcacatctggctccatgttgggccgaacagacaca
gccctcacaaacacctacagcgctctgccgcctatgcccagcttcaccatggcaaataacctgcctatgcaacccccagtc
cccagccagacctcctcatactcctgcatgctgcccaccagcccttcggtgaatgggcggagttatgatacctacaccccc
ccacatatgcagacacacatgaacagtcagccaatgggcacctcgggcacccacttcaacaggactcatttcccctggtgtg
tcagttccagttcaagttcccggaagtgaacctgatatgtctcaatactggccaagattacagtaa

SEQIDNO: 36(mPax6 nucleotide sequence)

atgcagaacagtcacagcggagtgaatcagcttggtggtgtctttgtcaacgggcggccactgccggactccaccc
ggcagaagatcgtagagctagctcacagcggggcccggccgtgcgacatttcccgaattctgcagacccatgcagatgc
aaaagtccaggtgctggacaatgaaaacgtatccaacggttgtgtgagtaaaattctgggcaggtattacgagactggctcc
atcagacccagggcaatcggagggagtaagccaagagtggcgactccagaagttgtaagcaaaatagcccagtataaac
gggagtgcccttccatctttgcttgggaaatccgagacagattattatccgaggggggtctgtaccaacgataacatacccagt
gtgtcatcaataaacagagttcttcgcaacctggctagcgaaaagcaacagatgggcgcagacggcatgtatgataaacta
aggatgttgaacgggcagaccggaagctggggcacacgccctggttggtatccggggacttcagtaccagggcaaccca
cgcaagatggctgccagcaacaggaaggaggggggagagaacaccaactccatcagttctaacggagaagactcggatg
aagctcagatgcgacttcagctgaagcggaagctgcaaagaaatagaacatcttttacccaagagcagattgaggctctgg
agaaagagtttgagaggacccattatccagatgtgtttgcccgggaaagactagcagccaaaatagatctacctgaagcaa
gaatacaggtatggttttctaatcgaagggccaaatggagaagagaagagaaactgaggaaccagagaagacaggcca
gcaacactcctagtcacattcctatcagcagcagcttcagtaccagtgtctaccagccaatcccacagcccaccacacctgt
ctcctccttcacatcaggttccatgttgggccgaacagacaccgccctcaccaacacgtacagtgctttgccacccatgccc
agcttcaccatggcaaacaacctgcctatgcaacccccagtccccagtcagacctcctcatactcgtgcatgctgcccacca
gcccgtcagtgaatgggcggagttatgatacctacacccctccgcacatgcaaacacacatgaacagtcagcccatgggc

acctcggggaccacttcaacaggactcatttcacctggagtgtcagttcccgtccaagttcccgggagtgaacctgacatgt
ctcagtactggcctcgattacagtaa

SEQIDNO: 37(hOtx2 amino acid sequence)

MMSYLKQPPYAVNGLSLTTSGMDLLHPSVGYPATPRKQRRERTTFTRAQ
LDVLEALFAKTRYPDIFMREEVALKINLPESRVQVWFKNRRAKCRQQQQQQQ
NGGQNKVRPAKKKTSPAREVSSESGTSGQFTPPSSTSVPTIASSSAPVSIWSPAS
ISPLSDPLSTSSSCMQRSYPMTYTQASGYSQGYAGSTSYFGGMDCGSYLTPMH
HQLPGPGATLSPMGTNAVTSHLNQSPASLSTQGYGASSLGFNSTTDCLDYKDQ
TASWKLNFNADCLDYKDQTSSWKFQVL

SEQIDNO: 38(mOtx2 amino acid sequence)

MMSYLKQPPYAVNGLSLTTSGMDLLHPSVGYPGPWASCPAATPRKQRRE
RTTFTRAQLDVLEALFAKTRYPDIFMREEVALKINLPESRVQVWFKNRRAKCR
QQQQQQQNGGQNKVRPAKKKSSPAREVSSESGTSGQFSPPSSTSVPTIASSSAP
VSIWSPASISPLSDPLSTSSSCMQRSYPMTYTQASGYSQGYAGSTSYFGGMDC
GSYLTPMHHQLPGPGATLSPMGTNAVTSHLNQSPASLSTQGYGASSLGFNSTT
DCLDYKDQTASWKLNFNADCLDYKDQTSSWKFQVL

SEQIDNO: 39(hOtx2 nucleotide sequence)

atgatgtcttatcttaagcaaccgccttacgcagtcaatgggctgagtctgaccacttcgggtatggacttgctgcacc
cctccgtgggctacccggccacccccggaaacagcgccgggagaggacgacgttcactcgggcgcagctagatgtgc
tggaagcactgtttgccaagacccggtacccagacatcttcatgcgagaggaggtggcactgaaaatcaacttgcccgagt
cgagggtgcaggtatggtttaagaatcgaagagctaagtgccgccaacaacagcaacaacagcagaatggaggtcaaaa
caaagtgagacctgccaaaaagaagacatctccagctcgggaagtgagttcagagagtggaacaagtggccaattcactc
cccctctagcacctcagtcccgaccattgccagcagcagtgctcctgtgtctatctggagcccagcttccatctcccactg
tcagatcccttgtccacctcctcttcctgcatgcagaggtcctatcccatgacctatactcaggcttcaggttatagtcaaggat
atgctggctcaacttcctactttggggggcatggactgtggatcatatttgacccctatgcatcaccagcttcccggaccaggg
gccacactcagtcccatgggtaccaatgcagtcaccagccatctcaatcagtccccagcttctctttccacccagggatatg
gagcttcaagcttgggttttaactcaaccactgattgcttggattataaggaccaaactgcctcctggaagcttaacttcaatgc
tgactgcttggattataaagatcagacatcctcgtggaaattccaggttttgtga

SEQIDNO:40(mOtx2 nucleotide sequence)

atgatgtcttatctaaagcaaccgccttacgcagtcaatgggctgagtctgaccacttcgggtatggacttgctgcatc
cctccgtgggctacccgggccctgggcttcttgtcctgcagccacccccggaaacagcgaagggagaggacgacatt
tactagggcacagctcgacgttctggaagctctgtttgccaagacccggtacccagacatcttcatgagggaagaggtggc
actgaaaatcaacttgccagaatccagggtgcaggtatggtttaagaatcgaagagctaagtgccgccaacagcagcagc
agcagcagaatggaggtcagaacaaagtgaggcctgccaagaagaagagctctccagctcgggaagtgagttcagaga

gtggaacaagtggccagttcagtcccccctctagtacctcagtcccaaccattgccagcagcagtgctccagtgtctatctg gagcccagcgtccatctccccactgtctgaccccttgtccacttcctcctcctgcatgcagaggtcctatcccatgacctatac tcaggcttcaggttatagtcaaggctatgctggctcaacttcctactttggggggcatggactgtggatcttatttgacccctatg catcaccagcttcctggaccaggggccacactcagtcccatgggtaccaatgctgttaccagccatctcaatcagtcccca gcttctctttccacccagggatatggagcttcaagcttgggttttaactcaaccactgattgcttggattataaggaccaaactg cctcttggaagcttaacttcaatgctgactgcttggattataaagatcagacgtcctcatggaaattccaggttttgtga

SEQIDNO: 41(SA-hAscl1 amino acid sequence)

MESSAKMESGGAGQQPQPQPQQPFLPPAACFFATAAAAAAAAAAAAAQ
SAQQQQQQQQQQQQQAPQLRPAADGQPSGGGHKSAPKQVKRQRSSAPELMR
CKRRLNFSGFGYSLPQQQPAAVARRNERERNRVKLVNLGFATLREHVPNGAA
NKKMSKVETLRSAVEYIRALQQLLDEHDAVSAAFQAGVLAPTIAPNYSNDLN
SMAGAPVSSYSSDEGSYDPLAPEEQELLDFTNWF

SEQIDNO: 42(hGFP nucleotide sequence)

taatcccacctccctctctgtgctgggactcacagagggagacctcaggaggcagtctgtccatcacatgtccaaat gcagagcataccctgggctgggcgcagtggcgcacaactgtaattccagcactttgggaggctgatgtggaaggatcact tgagcccagaagttctagaccagcctgggcaacatggcaagaccctatctctacaaaaaaagttaaaaaatcagccacgtg tggtgacacacacctgtagtcccagctattcaggaggctgaggtgaggggatcacttaaggctgggaggttgaggctgca gtgagtcgtggttgcgccactgcactccagcctgggcaacagtgagaccctgtctcaaaagacaaaaaaaaaaaaaaaaa aaaaagaacatatcctggtgtggagtaggggacgctgctctgacagaggctcggggggcctgagctggctctgtgagctgg ggaggaggcagacagccaggccttgtctgcaagcagacctggcagcattgggctggccgcccccccagggcctcctcttc atgcccagtgaatgactcaccttggcacagacacaatgttcggggtgggcacagtgcctgcttcccgccgcaccccagcc cccctcaaatgccttccgagaagcccattgagcaggggggcttgcattgcaccccagcctgacagcctggcatcttgggata aaagcagcacagcccccctaggggctgcccttgctgtgtggcgccaccggcggtggagaacaaggctctattcagcctgt gcccaggaaaggggatcaggggatgcccaggcatggacagtgggtggcaggggggggagaggagggctgtctgcttcc cagaagtccaaggacacaaatgggtgaggggactgggcaggttctgaccctgtgggaccagagtggagggcgtagat ggacctgaagtctccagggacaacagggcccaggtctcaggctcctagttgggcccagtggctccagcgtttccaaaccc atccatccccagaggttcttcccatctctccaggctgatgtgtgggaactcgaggaaataaatctccagtgggagacggag gggtggccagggaaacggggcgctgcaggaataaagacgagccagcacagccagctcatgtgtaacggctttgtggag ctgtcaaggcctggtctctgggagagaggcacagggaggccagacaaggaaggggtgacctggagggacagatccag gggctaaagtcctgataaggcaagagagtgccggcccccctcttgccctatcaggacctccactgccacatagaggccatg attgacccttagacaaagggctggtgtccaatcccagccccagccccagaactccagggaatgaatgggcagagagca ggaatgtgggacatctgtgttcaagggaaggactccaggagtctgctgggaatgaggcctagtaggaaatgaggtggccc ttgagggtacagaacaggttcattcttcgccaaattcccagcaccttgcaggcacttacagctgagtgagataatgcctgggt tatgaaatcaaaaagttggaaagcaggtcagaggtcatctggtacagcccttccttccctttttttttttttttttttgtgagacaagg tctctctctgttgcccaggctggagtggcgcaaacacagctcactgcagcctcaacctactgggctcaagcaatcctccagc ctcagcctcccaaagtgctgggattacaagcatgagccaccccactcagcccttttcctttttttaattgatgcataataattgt

aagtattcatcatggtccaaccaacccttttcttgacccaccttcctagagagagggtcctcttgcttcagcggtcagggcccc agacccatggtctggctccaggtaccacctgcctcatgcaggagttggcgtgcccaggaagctctgcctctgggcacagt gacctcagtgggggtgaggggagctctccccatagctgggctgcggcccaacccccaccccctcaggctatgccaggggggt gttgccaggggcacccgggcatcgccagtctagcccactccttcataaagccctcgcatcccaggagcgagcagagcca gagcaggttggagaggagacgcatcacctccgctgctcgcgg

SEQIDNO: 43(shorthGFP nucleotide sequence)

aacatatcctggtgtggagtaggggacgctgctctgacagaggctcgggggcctgagctggctctgtgagctggg gaggaggcagacagccaggccttgtctgcaagcagacctggcagcattgggctggccgcccccccagggcctcctcttca tgcccagtgaatgactcaccttggcacagacacaatgttcggggtgggcacagtgcctgcttcccgccgcacccccagccc ccctcaaatgccttccgagaagcccattgagcaggggggcttgcattgcaccccagcctgacagcctggcatcttgggataa aagcagcacagccccctaggggctgcccttgctgtgtggcgccaccggcggtggagaacaaggctctattcagcctgtg cccaggaaaggggatcaggggatgcccaggcatggacagtgggtggcaggggggggagaggagggctgtctgcttccc agaagtccaaggacacaaatgggtgaggggagagctctccccatagctgggctgcggcccaacccccaccccctcaggc tatgccaggggggtgttgccaggggcacccgggcatcgccagtctagcccactccttcataaagccctcgcatcccaggag cgagcagagccagagcaggttggagaggagacgcatcacctccgctgctcgcgg

SEQIDNO: 44(VP16 amino acid sequence)
MLGDGDSPGPGFTPHDSAPYGALDMADFEFEQMFTDALGIDEYGG

SEQIDNO: 45(VP16 nucleotide sequence)

atgttggggggacggggattccccgggtccgggatttacccccccacgactccgccccctacggcgctctggatatg gccgacttcgagtttgagcagatgtttaccgatgcccttggaattgacgagtacggtggg

SEQIDNO: 46(SV40 nucleotide sequence)

Cgatggagcggagaatgggcggaactgggcggagttaggggcgggatgggcggagttaggggcgggactat ggttgctgactaattgagatgcatgctttgcatacttctgcctgctggggagcctggggactttccacacctggttgctgacta attgagatgcatgctttgcatacttctgcctgctggggagcctggggactttccacaccctaactgacacacattccacagc

General method

Human glioma cell culture

Human glioma cell lines U251 and U87 (purchased from the cell bank of Shanghai Institute of Life Sciences, Chinese Academy of Sciences) were cultured in a 37 °C incubator containing 5% $CO_2$. The culture medium was DMEM medium containing 10% fetal bovine serum and 1% penicillin/streptomycin. Add lentivirus, change the solution into inducing medium (DMEM, 2% B-27, 1% PS) 12 hours after infection, and then change the solution into nerve medium DMEM/F-12, 2% B-27, 1% PS, 20 ng/ml BDNF, 20 ng/ml GDNF after 48 hours. Change half of the culture medium every three days.

Immunostaining

The immunostaining of cultured cells was carried out according to "Direct conversion of fibroblasts to functional neurons by defined factors" (Vierbuchen, T. et al. Nature 463, 1035-1041 (2010)). The immunostaining test of tissue sections was carried out according to the published methods. The first antibody used in immune coloration includes: mouse anti-NeuN (Millipore, 1:100), rabbit anti-Dsred (Clontech, 1:500), mouse anti-Tuj 1 (Covance, 1:500), mouse anti-Map2 (Sigma, 1:500), rabbit anti-GFP (Invitrogen, 1: 1000), chip anti-GFP (Invitrogen, 1: 1000), rabbit anti-Synapsin I (Millipore, 1:1000), rabbit anti - VGLUT 1 (Synaptic Systems, 1:500), rabbit anti-Ki67 (1:200; RM-9106; Thermo Fisher Scientific), mouse anti-BrdU(1:200; B2531; Sigma)。FITC -, Cy3 - and Cy5 - coupled secondary antibodies were purchased from Jackson Immunoresearch.

BrdU(5-bromodeoxyuracil nucleoside) labeling and cell proliferation experiment

The cultured human glioma cells were added with 10 mM BrdU (Sigma) and incubated for 2 hours or continuously according to the experimental requirements. The color of BrdU was detected by anti-BrdU antibody. The proliferating cells were also tested with Ki67 antibody. In addition, when evaluating the growth of glioma in 24 orifice plate ($5 \times 10^4$ cells/well), count the number of cells at different time points (days 0, 3, 7, 14 and 21).

Glial cell trans-differentiation model in vitro

(1) Plasmid construction and virus infection

On the vector template of FUGW-IRES-EGFP (refer to the document Efficient transfer, integration, and sustained long term expression of the transgene in adult rat brain injected with a lentiviral vector. Proc Natl Acad Sci U S A 93: 11382 - 11388 for vector information), replace the CAG promoter with the cloned human NG2 promoter, and then construct the polynucleotide fragment of the transcription factor onto the lentivirus vector to generate hNG2-transcription factor-IRES-EGFP lentivirus plasmid. The packaging of lentivirus refers to the literature "Production and purification of lentivirus vectors" (Tiscornia, G., Singer, O.& Verma, I. M. Nat. Protocol. 1, 241-245 (2006)). The lentivirus was added to NG2 cells after 24 hours of plate culture, and the culture medium was changed after 24 hours of infection: DMEM/F12, B27, Glutamax and penicillin/streptomycin. After 6-7 days of infection, brain-derived neurotrophic factor (BDNF; Pepro-Tech, 20ng/ml) was added to the culture medium every three days.

(2) NG2 cells differentiate into neurons

Most of the cultured mouse NG2 cells were immuno-positive to NG2 glial cell marker NG2, and a small number of cells expressed oligodendrocyte marker molecules O4 and CNPase, but no expression of neuron marker molecule Tuj 1 and stem cell marker molecules Sox2 and Oct4 was detected. After 10 days of transfection of NG2 cells with hNG2 transcription factor-IRES-GFP lentivirus, the morphology of NG2 cells and the marker molecule Tuj 1 of neurons were detected. After 21 days of infection with lentivirus, the marker molecules NeuN and MAP2 of mature neurons were detected at the same time, and whether the cells with neuron morphology and positive markers could produce action potential by electrophysiological recording. If spontaneous postsynaptic current could be recorded, it means that these neurons could form functional synapses.

(3) Neurons induced by NG2 cells can survive after transplantation into vivo

Whether the transdifferentiated neurons induced in vitro can survive and function in vivo is the key to whether they can be used for disease treatment. Two weeks after the neurons induced by NG2 cells are transplanted into the cerebral cortex, the immunohistochemical experiment of the transcription factor needs to observe whether the transplanted cells can attach to the edge of the cortex, and detect whether the cells form neurites and extend deeper into the cortex. At the same time, immunofluorescence co-localization test was used to detect whether the transplanted cells expressed neuronal marker molecules Tuj 1, NeuN and MAP2.

It should be noted that, except for NG2 promoter, other glial cell-specific promoters can achieve similar trans-differentiation functions. Although there are slight differences in transformation efficiency, for uniform screening, the in vitro model of glial cell trans-differentiation is uniformly carried out under the same vector type and the same promoter.

In vivo model of glial cell transdifferentiation

(1) Construction of adeno-associated virus plasmid and virus infection

The GFAP promoter was cloned on the vector template of AAV-FLEX-Arch-GFP (Addgene, # 22222) to replace the CAG promoter and retain the CMV enhancer. The AAV-mCherry plasmid (control group) was obtained after replacing GFP with the mCherry coding frame. The transcription factor was cloned into the AAV-mCherry plasmid to obtain the AAV-mNeurog2/mCherry plasmid. The target gene can specifically target astrocytes under the action of GFAP promoter.

(2) Astrocytes differentiate into neurons

The virus AAV-mCherry or AAV-transcription factor/mCherry was injected into one side of the tectum of adult wild-type mice, and then the brain tissue samples were collected at different time points. On the 3rd and 30th day of virus injection, observe whether the mCherry of control virus AAV-mCherry and mice with virus AAV-transcription factor/mCherry co-located with NeuN. In order to prove that the induced neurons are functional and active neurons, physiological recording of the midbrain electroencephalogram (MEG) of AAV virus infection was performed. Record the inward Na+current and outward K+current in the voltage clamp mode, count the proportion of recorded cell action potential and postsynaptic currents, and observe whether the postsynaptic current signal disappears and whether the post-synaptic current signal appears after elution through the blocker NBQX to determine whether the induced neurons integrate into the neural circuit to establish synaptic connection, To determine whether it is a functional neuron.

The AAV virus is carried out with reference to the mouse brain atlas. After the injection of the virus, the midbrain and spinal cord of the back were collected at different time points for immunocoloration or brain slice recording. The injection concentration and speed of intact spinal cord and injured spinal cord virus are consistent with the injection volume per needle and the brain area. The injection is conducted in the spinal cord at an angle of 30 °.

Nerve injury repair model

(1) Spinal cord injury and virus transfection

The T8-T10 model of complete spinal cord injury in mice was constructed (referring to the method of McDonough A, Monterubio A, Arizona J, et al. Calibrated Forceps Model of Spinal Cord Compression Injury. Jove-Journal of Visualized Experiments 2015.). AAV-mCherry virus and AAV-transcription factor/mCherry were injected into both sides of the injured spinal cord immediately after injury. Observe whether mCherry and NeuN are co-located 3 days after virus injection and 30 days after virus injection.

(2) Repair detection of spinal cord injury

After thoracic spinal cord injury, the loss of sensory afferent leads to the weakening of the inhibitory effect of the descending inhibitory system of the brain stem, which leads to the over-sensitivity of the tail to external stimuli. We used the tail flick experiment model to test the sensory ability of mice by measuring the delay time of the tail response of two groups of mice under 48 °C and 52 °C thermal stimulation. The motor function of mice was scored according to the BMS standard. For the test method, refer to Basso Mouse Scale for localization detection differences in recovery after final core adjustment in five common mouse strains J Neurotrauma, 2006. 23 (5): p. 635-59.

Glioma model

The mice used for glioma model transplantation were NOD-scid mice of seven weeks. Provide human glioma cells with 0.25% trypsin digestion and induction for 3 days or without induction. Centrifuge and remove the supernatant to make the cell density after concentration about $2.5 \times 10^5$ cells/ $\mu$L. Transplant brain striatum of each mouse 2 $\mu$L (5 in total $\times 10^5$ cells). Histochemistry was performed 3 weeks after transplantation or virus was injected one week after transplantation, followed by immunohistochemistry.

Examples

Example 1: Functional fragment of single transcription factor promotes glial cells to differentiate into neurons

First of all, we used the model of glial cell trans-differentiation in vitro to conduct preliminary screening, and obtained a batch of transcription factors that can induce glial cell trans-differentiation into neurons. The coding sequence and conversion efficiency of the transcription factors used are shown in Table 1.

In vitro trans-differentiation efficiency% = (the number of virus-infected fluorescence-positive cells with positive neuronal marker Tuj1 and spontaneous postsynaptic current detected by electrophysiology/the total number of virus-infected fluorescence-positive cells) $\times$ 100%, at least 100 transdifferentiated cells with Tuj1 positive and spontaneous postsynaptic current can be detected for each transcription factor on average.

Table 1 Glial cell trans-differentiation efficiency of single transcription factor in vitro

| transcription factor | Human (wild type) | Trans-differentiation efficiency | Mouse (wild type) | Trans-differentiation efficiency |
|---|---|---|---|---|
| NeuroD1 | SEQ ID NO: 1/3 | 42.3% | SEQ ID NO: 2/4 | 45.8% |
| Brn2 | SEQ ID NO: 5/7 | 8.7% | SEQ ID NO: 6/8 | 9.6% |
| Ascl1 | SEQ ID NO: 9/11 | 68.5% | SEQ ID NO: 10/12 | 67.3% |
| Ngn2 | SEQ ID NO: 13/15 | 51.2% | SEQ ID NO: 14/16 | 46.3% |
| Gsx1 | SEQ ID NO: 17/19 | 6.2% | SEQ ID NO: 18/20 | 6.9% |
| Tbr1 | SEQ ID NO: 21/23 | 4.2% | SEQ ID NO: 22/24 | 5.3% |
| Dlx2 | SEQ ID NO: 25/27 | 32.1% | SEQ ID NO: 26/28 | 35.7% |
| Ptf1a | SEQ ID NO: 29/31 | 11.6% | SEQ ID NO: 30/32 | 15.3% |
| Pax6 | SEQ ID NO: 33/35 | 25.2% | SEQ ID NO: 34/36 | 19.8% |
| Otx2 | SEQ ID NO: 37/39 | 6.9% | SEQ ID NO: 38/40 | 8.4% |

Both human and mouse derived transcription factors have the ability to differentiate glial cells into neurons in vitro.

In further research, we also found that for human Ascl1 protein, if the five conserved serine-proline (SP) phosphorylation sites (located at positions 93, 190, 194, 207 and 223 of the protein sequence) of the protein sequence were mutated into alanine-proline (AP) (enhanced Ascl1 (SA-hAscl1), and the protein sequence SEQ ID NO: 41), the transformation efficiency could be further improved to 85.5%.

Example 2 Functional fragment of single transcription factor promotes glial cell trans-differentiation in the dorsal midbrain

According to the in vivo model of glial cell trans-differentiation, we tried to use the selected transcription factors in Example 1 to induce glial cells in the dorsal midbrain, as shown in the following table (Table 2). Different transcription factors showed significantly different transformation efficiency.

The efficiency of trans-differentiation in vivo is characterized by the proportion of the occurrence of neuronal co-localization. The efficiency of trans-differentiation in vivo % = (the number of virus-infected fluorescent positive cells with positive neuronal marker NeuN and spontaneous postsynaptic current can be detected by electrophysiology/the total number of virus-infected fluorescent positive cells) × 100%, at least 100 transdifferentiated cells with NeuN positive and spontaneous postsynaptic current can be detected for each transcription factor on average.

Table 2 Glial cell trans-differentiation efficiency of single transcription factor in vivo (sequence as shown in Example 1)

| | Human Transcription Factor | | Murine transcription factor | |
|---|---|---|---|---|
| | Neuronal co-location | Whether synapses are formed | Neuronal co-location | Whether synapses are formed |
| NeuroD1 | 66.4% | yes | 68.7% | yes |
| Brn2 | 8.4% | yes | 9.3% | yes |
| Ascl1 | 72.8% | yes | 79.1% | yes |
| Ngn2 | 71.2% | yes | 73.5% | yes |
| Gsx1 | 5.1% | yes | 5.7% | yes |

(continued)

| | Human Transcription Factor | | Murine transcription factor | |
|---|---|---|---|---|
| | Neuronal co-location | Whether synapses are formed | Neuronal co-location | Whether synapses are formed |
| Tbr1 | 4.4% | yes | 4.8% | yes |
| Dlx2 | 23.6% | yes | 29.4% | yes |
| Ptf1a | 8.2% | yes | 11.3% | yes |
| Pax6 | 21.2% | yes | 24.8% | yes |
| Otx2 | 7.2% | yes | 8.8% | yes |
| Enhanced Ascl 1 | 86.6% | yes | 83.6% | yes |

Based on the in vivo model, we further studied the expression elements of AAV expression vector in detail, and found at least three technical improvements that can significantly increase the efficiency of glial cell transformation.

(1) Insertion of VP16 fusion protein

VP16 is the active domain of VP16 protein from Herpes simplex virus (SEQ ID NO: 45). We cloned its gene sequence into AAV-transcription factor/mCherry plasmid to obtain AAV-VP16-transcription factor/mCherry plasmid. VP16 can be single or multiple strings. The plasmid will translate the fusion protein VP16-transcription factor, thus enhancing the function of activating gene expression of the transcription factor. The efficiency of neurons induced by AAV-VP16-transcription factor/mCherry is significantly higher, and the induction speed is faster (see Table 3).

(2) Promoter shortening

After changing the human hGFAP promoter 2.2 kb (SEQ ID NO: 42) to 683 bp (SEQ ID NO: 43), we found that the modified promoter did not affect the specificity of targeted astrocytes, at the same time, it improved the packaging efficiency of AAV, reduced the empty shell rate of virus packaging, and obtained a higher and purer AAV titer. When induced in vivo, Short-hGFAP-AAV transcription factor/mCherry can improve the efficiency of virus transfection, reduce the number of cell death, and make the induction process safer (see Table 3).

(3) Enhancer insertion

We inserted the enhancer (SEQ ID NO: 46) of simian vacuolating virus 40 SV40 into the hGFAP-AAV transcription factor/mCherry plasmid to obtain SV40-hGFAP-AAV transcription factor/mCherry. The enhancer of SV40 can greatly enhance the activity of hGFAP promoter, so that the target gene can be efficiently expressed in vivo, thus improving the efficiency of neuron induction (see Table 3).

The above three methods of enhancing expression can be used alone or in combination or at the same time, which can improve the inducing efficiency of the transcription factors described in this embodiment (taking human Ascl1 as an example, see Table 3).

Table 3 Improvement of Ascl 1 conversion efficiency

| transcription factor | sequence | VP16 insertion | Promoter truncation | SV40 insertion |
|---|---|---|---|---|
| wild type hAscl1 | SEQ ID NO: 9 | 75.8% | 77.2% | 76.4% |
| Enhanced hAscl1 | SEQ ID NO: 41 | 88.4% | 89.2% | 88.9% |

Similarly, for other transcription factors, any of the above three technical solutions can significantly improve the transformation efficiency or AAV titer. Table 4 shows the average transformation efficiency of other transcription factors after using the above transformation strategies.

Table 4 Effect of transformation strategy of expression vector on trans-differentiation efficiency

| transcription factor | sequence | VP16 insertion | Promoter truncation | SV40 insertion |
|---|---|---|---|---|
| hNgn2 | SEQ ID NO: 13 | 73.8% | 74.2% | 74.4% |
| hNeuroD1 | SEQ ID NO: 1 | 68.3% | 69.6% | 68.5% |
| hDlx2 | SEQ ID NO: 25 | 25.3% | 26.3% | 25.4% |

Example 3 The combination of functional fragments of transcription factors further increases the efficiency of glial cell trans-differentiation

According to the trans-differentiation model of glial cells in vitro and in vivo, and in combination with the vector transformation strategy described in Example 2, we first expand the selected transcription factors into random pairwise combinations. Among them, different transcription factors can be expressed simultaneously in the same vector or separately in different expression vectors. The expression ratio in the following table is the molar concentration ratio of the functional protein expressed in the actual study. In NeuroD1, Brn2, Gsx1, Tbr1, Dlx2, Ptf1a, Pax6, Otx2 transcription factors, we unexpectedly obtained several groups of single transcription factors with low efficiency, but when combined, they can significantly improve the efficiency of transcription factors. Moreover, the closer the molar concentration ratio of the expressed functional protein is, the higher the conversion efficiency obtained (see Table 5).

Table 5 Transcription factor combination can improve the efficiency of glial cell trans-differentiation

| (Trans-differentiation in vitro model, sequence selected from human sequence in Example 1) | | | | | |
|---|---|---|---|---|---|
| Factor combination | | Expression scale | | Time required to achieve induction of 50% cell trans-differentiation (unit: day) | Trans-differentiation efficiency (calculation method is the same as Example 1) |
| Factor A | Factor B | Factor A | Factor B | | |
| NeuroD1 | Brn2 | 1 | 1 | 11.2 | 76.2% |
| | | 2 | 1 | 13.6 | 71.5% |
| Gsx1 | Tbr1 | 1 | 1 | 12.4 | 63.2% |
| | | 2 | 1 | 14.7 | 56.9% |
| Dlx2 | Ptf1a | 1 | 1 | 11.7 | 69.2% |
| | | 3 | 1 | 12.8 | 61.2% |
| Pax6 | Otx2 | 1 | 1 | 12.3 | 63.8% |
| | | 2 | 1 | 13.9 | 54.3% |

Taking NeuroD1 and Brn2 as examples, the trans-differentiation efficiency of single use was 42.30% and 8.70% respectively (Table 1), while the trans-differentiation efficiency of combined use (1:1) was 76.2%, with synergistic effect. Similarly, Gsx1+Tbr1, Dlx2+Ptfla, Pax6+Otx2 can also synergistically significantly improve the efficiency of trans-differentiation.

In the study, we also found that Ascl1 and Ngn2 are two key transcription factors. When combined with any of the above transcription factors or transcription factors, they can achieve significant enhancement of transcription efficiency, and achieve the function of superposition or synergy (see Tables 6 and 7).

Table 6 The key transcription factor Ascl1 can significantly enhance the efficiency of glial cell trans-differentiation (trans-differentiation in vitro model, sequence selected from human sequence in Example 1)

| Key factors | Factor combination | | Expression scale | | | Trans-differentiation efficiency (calculation method is the same as Example 1) |
|---|---|---|---|---|---|---|
| Factor A | Factor B | Factor C | Factor A | Factor B | Factor C | |
| Ascl1 | NeuroD1 | / | 1 | 4 | / | 82.2% |
| | / | Brn2 | 1 | / | 2 | 81.5% |
| | NeuroD1 | Brn2 | 1 | 2 | 2 | 83.6% |
| | NeuroD1 | Brn2 | 1 | 1 | 1 | 83.7% |
| | NeuroD1 | Brn2 | 2 | 1 | 1 | 85.8% |
| Ascl1 | Gsx1 | / | 1 | 1 | / | 81.5% |
| | / | Tbr1 | 2 | / | 1 | 80.2% |
| | Gsx1 | Tbr1 | 2 | 1 | 1 | 83.5% |
| Ascl1 | D1x2 | / | 4 | 1 | / | 82.8% |
| | / | Ptf1a | 2 | / | 1 | 81.4% |
| | D1x2 | Ptf1a | 1 | 1 | 1 | 84.2% |
| Ascl1 | Pax6 | / | 1 | 1 | / | 78.5% |
| | / | Otx2 | 1 | / | 2 | 76.8% |
| | Pax6 | Otx2 | 1 | 2 | 2 | 80.3% |
| Ascl1 | Ngn2 | / | 1 | 1 | / | 91.5% |
| | Ngn2 | / | 4 | 1 | / | 86.3% |
| | Ngn2 | / | 1 | 4 | / | 85.4% |

Table 7 Key transcription factor Ngn2 can significantly enhance the efficiency of glial cell trans-differentiation (Trans-differentiation in vitro model, sequence selected from human sequence in Example 1)

| Key factors | Factor combination | | Expression scale | | | Trans-differentiation efficiency |
|---|---|---|---|---|---|---|
| Factor A | Factor B | Factor C | Factor A | Factor B | Factor C | |
| Ngn2 | NeuroD1 | / | 1 | 4 | / | 78.5% |
| | NeuroD1 | / | 1 | 1 | / | 88.6% |
| | NeuroD1 | / | 4 | 1 | / | 75.6% |
| | / | Brn2 | 1 | / | 2 | 79.6% |
| | NeuroD1 | Brn2 | 1 | 2 | 2 | 82.3% |
| | NeuroD1 | Brn2 | 1 | 1 | 1 | 85.4% |
| | NeuroD1 | Brn2 | 2 | 1 | 1 | 88.2% |
| Ngn2 | Gsx1 | / | 1 | 1 | / | 76.3% |
| | / | Tbr1 | 2 | / | 1 | 77.9% |
| | Gsx1 | Tbr1 | 2 | 1 | 1 | 80.3% |
| Ngn2 | D1x2 | / | 4 | 1 | / | 75.4% |
| | / | Ptf1a | 2 | / | 1 | 76.3% |
| | D1x2 | Ptf1a | 1 | 1 | 1 | 79.8% |

(continued)

| (Trans-differentiation in vitro model, sequence selected from human sequence in Example 1) | | | | | | |
|---|---|---|---|---|---|---|
| Key factors | Factor combination | | Expression scale | | | Trans-differentiation efficiency |
| Factor A | Factor B | Factor C | Factor A | Factor B | Factor C | |
| Ngn2 | Pax6 | / | 1 | 1 | / | 75.1% |
| | / | Otx2 | 1 | / | 2 | 76.3% |
| | Pax6 | Otx2 | 1 | 2 | 2 | 77.8% |

Example 4 Application of transcription factor and its combination in the repair of spinal cord injury

Spinal cord injury (SCI) is a kind of central nervous system injury disease, accompanied by the death of spinal cord neurons and the formation of glial scar. In vivo neuronal reprogramming converts astrocytes into neurons, which may alleviate the damage caused by SCI, and is expected to become a new treatment method.

According to the nerve injury repair model, we found that the transcription factor or combination of transcription factors described in Examples 1-3 with conversion efficiency of more than 50% have the ability to induce glial cells at the injured site to obtain electrophysiological characteristics, and can accept external signal input. According to the detection model of spinal cord injury. We found that these transcription factor reprogramming neurons are very helpful for the recovery of sensory function and motor function of mice with spinal cord injury, especially the transcription factor or combination of transcription factors with conversion efficiency of more than 75% described in Examples 1-3. The preferred transcription factors and their combinations are shown in Table 8.

Table 8 Preferred transcription factors and their combinations for nerve injury repair

| Key factors | Factor combination | | Expression scale | | |
|---|---|---|---|---|---|
| Factor A | Factor B | Factor C | Factor A | Factor B | Factor C |
| Ascl1 | / | / | 1 | / | / |
| | Ngn2 | / | 1 | 1 | / |
| | D1x2 | Ptf1a | 1 | 1 | 1 |
| | D1x2 | / | 2 | 1 | / |
| | NeuroD1 | / | 2 | 1 | / |
| Ngn2 | / | / | 1 | / | / |
| | NeuroD1 | Brn2 | 1 | 1 | 1 |
| | NeuroD1 | / | 1 | 1 | / |
| / | NeuroD1 | Brn2 | / | 1 | 1 |

Example 5 Combination of functional fragments of transcription factor trans-differentiation into neurons in glioma cells in vitro

On the basis of obtaining the transcription factors and their combinations as shown in Example 1-3, we also explored the application of transcription factors or combinations of transcription factors with high conversion efficiency to promote the trans-differentiation of glioma cells into neurons. The implementation scheme is similar to the model of glioblast trans-differentiation in vivo or in vitro. Taking the factor combination of NeuroD1 and Brn2 as an example, the specific implementation is as follows:

(1) Plasmid construction and virus infection

On the vector template of FUGW-IRES-EGFP (refer to the document Efficient transfer, integration, and sustained long term expression of the transgene in adult rat brain injected with a lentiviral vector. Proc Natl Acad Sci U S A 93:11382 - 11388 for vector information), the polynucleotide functional fragment was constructed onto the lentivirus vector to generate lentivirus plasmid carrying the polynucleotide functional fragment. In one embodiment, a human NeuroD1 transcription factor fragment (SEQ ID NO: 3) was constructed onto the lentivirus vector to generate hNeuroD1 IRES EGFP lentivirus plasmid. The packaging of lentivirus refers to the literature "Production and purification of lentivirus vectors" (Tiscornia, G., Singer, O.&Verma, I. M. Nat. Protocol. 1, 241-245 (2006)).

The lentivirus was added to the human glioma cells after 24 hours of plate culture, and the culture medium was changed after 24 hours of infection: DMEM/F12, B27, Glutamax and penicillin/streptomycin. After 6-7 days of infection, brain-derived neurotrophic factor (BDNF; PeproTech, 20ng/ml) was added to the culture medium every three days.

(2) NeuroD1 converts glioma cells into neurons

After the cultured human glioma cell U251 was infected with hNeuroD1-IRES-EGFP lentivirus for 14 days, we found that part of Tuj1 positive cells appeared and showed the morphology of neurons (Fig. 1B, D), indicating that NeuroD1 alone can transform glioma cell U251 into neurons, with a conversion efficiency of 5.1%.

(3) Co-expression of NeuroD1 and Brn2 improves the efficiency of glioma cells to differentiate into neurons

Although NeuroD1 alone can transform glioma cells into neurons, its induction efficiency is not very high. In order to improve the induction efficiency, we tested other transcription factors, and found that NeuroD 1 and Brn2 (SEQ ID NO: 7) together could very efficiently transform glioma cell U251 into neurons, and the cells showed the morphology of mature neurons (Fig. 1C, D), and the conversion efficiency was 58.3%. We also tested another human glioma cell U87, and found that NeuroD1 and Brn2 can also transform glioma cell U87 into neurons very efficiently, with a conversion efficiency of 61.5%.

(4) Molecular expression properties of glioma cells transdifferentiated neurons

21 days after glioma cell U251 was infected with lentivirus hNeuroD1-IRES-EGFP and hBrn2-IRES-EGFP, cellular immunofluorescence showed that the induced neurons expressed the marker molecules MAP2 (Fig. 2A) and synapsin I (Fig. 2B-D) of mature neurons, and expressed the marker molecule VGLUT1 (Fig. 2E-H) of glutamate neurons, indicating that the induced neurons were mainly excitatory neurons.

(5) Electrophysiological Properties of Glioma Transdifferentiated Neurons

After glioma cell U251 was infected with lentivirus hNeuroD1-IRES-EGFP and hBrn2-IRES-EGFP for 28 days, electrophysiological records showed that the induced neurons could emit multiple action potentials (Fig. 3A, B), and the postsynaptic current signals of the induced neurons could be detected (Fig. 3C). After the addition of blockers CNQX and AP5, the postsynaptic current signals disappeared, indicating that the induced neurons could receive synaptic signals and were functional neurons.

(6) NeuroD1 and Brn2 induced trans-differentiation led to the withdrawal of glioma cells from the cell cycle

Neuron cells are cells that withdraw from the cell cycle and no longer divide. NeuroD1 and Brn2 can induce glioma cells into neurons, which will cause glioma cells to withdraw from the cell cycle. To further confirm this point, we labeled BrdU at different time periods (the 1st, 3rd and 5th days) of lentivirus infection, and then immunocytochemical analysis was performed 2 hours after labeling (Fig. 4A). Compared with the control group, the ratio of BrdU positive number of glioma cells expressed by NeuroD1 and Brn2 lentivirus decreased sharply (Fig. 4B), which suggested that NeuroD1 and Brn2 mediated neuronal reprogramming led to the withdrawal of glioma cells from the cell cycle. In addition, after 5 days of lentivirus infection, continuous BrdU labeling was performed until 14 days. Immunocytochemical analysis showed that the number of BrdU positive glioma cells expressed by NeuroD1 and Brn2 lentivirus was significantly reduced (Figure 4C-E).

(7) NeuroD1 and Brn2 induced trans-differentiation inhibits the proliferation of glioma cells

Further, we performed immunofluorescence staining on the endogenous molecular marker Ki67 as a proliferating cell, and found that the number of Ki67 positive glioma cells expressed by NeuroD1 and Brn2 lentivirus was significantly reduced (Fig. 5A, B). We made quantitative statistics on the number of cells after virus infection for different times, and found that the growth of glioma cells infected with NeuroD1 and Brn2 lentivirus reached a stable state on the 7th day, and no longer increased significantly (Fig. 5C).

These results together show that NeuroD1 and Brn2 can induce malignant proliferating glioma cells into terminally differentiated neurons, and cause glioma cells to withdraw from the cell cycle, thus no longer proliferate.

(8) The tumorigenicity of glioma cells expressing NeuroD1 and Brn2 was significantly reduced in vivo

Because NeuroD1 and Brn2 can induce glioma cells cultured in vitro into neurons and cause glioma cells to withdraw from the cell cycle, the ability of these induced glioma cells to generate tumors in vivo will also be affected. We have carried out an in situ tumor cell transplantation experiment. The human glioma cell U251 cells infected with NeuroD1 and Brn2 lentivirus for 3 days ($5 \times 105$) Transplanted into the striatum of NOD-scid mice, the size of tumor tissue volume was evaluated 21 days later. The results showed that compared with the control, the tumor tissue of glioma cells infected by NeuroD1 and Brn2 lentivirus was significantly smaller, indicating that the tumorigenicity of these glioma cells was significantly reduced.

Then, we selected transcription factors or combinations of transcription factors with a transformation efficiency of more than 50% to test the trans-differentiation ability of these groups of transcription factors in glioma cells and the inhibition ability of the proliferation of glioma cells, and found that transcription factors or combinations of transcription factors with a trans-differentiation efficiency higher than 75% have the best inhibition effect on glioblast-derived tumors. The preferred transcription factors and their combinations are shown in Table 9.

Table 9 Preferred transcription factors and their combinations that inhibit glioma cells

| Key factors | Factor combination | | Expression scale | | |
|---|---|---|---|---|---|
| Factor A | Factor B | Factor C | Factor A | Factor B | Factor C |
| Ascl1 | / | / | 1 | / | / |
| | Ngn2 | / | 1 | 1 | / |
| | NeuroD1 | Brn2 | 1 | 1 | 1 |
| | D1x2 | / | 2 | 1 | / |
| | NeuroD1 | / | 2 | 1 | / |
| Ngn2 | / | / | 1 | / | / |
| | Gsx1 | Tbr1 | 1 | 1 | 1 |
| | NeuroD1 | / | 1 | 1 | / |
| / | NeuroD1 | Brn2 | / | 1 | 1 |

Example 6 The combination of transcription factor functional fragments inhibits the growth of glioma cells in the brain through reprogramming in vivo

On the basis of Example 5, we tried to verify the effect of transcription factors on the mouse transplanted tumor model, still taking the combination of NeuroD 1 and Brn2 as an example, the specific implementation is as follows:

(1) Construction of AAV plasmid

On the vector template of AAV-FLEX-Arch-GFP (Addgene, # 22222), the fragment from human NeuroD1 (SEQ ID No.: 3) was constructed onto the vector to obtain AAV-hNeuroD1-P2A-GFP. P2A is a self-cleaving polypeptide, which can achieve high co-expression of hNeuroD1 and GFP. The CDS (SEQ ID No.: 7) fragment from human Brn2 gene was constructed onto the vector to obtain AAV-hBrn2-P2A-GFP.

(2) AAV vector of NeuroD1 and Brn2 inhibits the growth of glioma cells in the brain

In order to confirm whether NeuroD1 and Brn2 induced reprogramming has the ability to treat glioma cells, we first carried out intracerebral transplantation of glioma cells ($5 \times 10^5$). Seven days after transplantation, we injected the AAV vector of NeuroD 1 and Brn2 in situ. After 30 days of virus injection, immunohistochemistry analysis showed that the cells infected with the virus expressed neural marker molecule Tuj 1, showing the morphology of neurons. At the same time, the volume of tumor also decreased significantly. More importantly, the life span of mice injected with NeuroD1 and Brn2 AAV virus was significantly prolonged.

Other transcription factors or combinations of transcription factors with trans-differentiation efficiency higher than 75% have also been observed in the glioma inhibition model.

(3) AAV vector expressing NeuroD1 and Brn2 inhibits the growth of tumor cells in human glioma U87 BALB/CA-nu mice heterotopic inoculation model

Cultured human U87 human glioma cells were inoculated into the armpit of nude mice during the logarithmic growth phase and passed through two passages. The tumor-bearing mice were taken under aseptic conditions, cut the tumor into small pieces of rice grains with uniform size, and inoculated subcutaneously into the armpit of nude mice with the insertion needle. After the tumor grew to about 100mm$^3$, the nude mice with appropriate tumor were randomly divided into groups, and the drug administration began after grouping. All samples were dissolved with PBS, and the intratumoral injection volume was 50 μL/tumor, measure the length and width of the tumor every 3 days, and calculate the tumor volume with the following formula:

$$\text{Volume}=(\text{length} \times \text{Width}^2)/2$$

The tumor inhibition rate is calculated according to the following formula:

$$\text{Tumor inhibition rate\%}=(\text{V model group} - \text{V administration group})/\text{V model group} \times 100\%$$

The animals were killed in the later stage, and the tumor was weighed for biochemical and molecular detection. The results showed that the expression of AAV-mediated reprogramming factor significantly reduced the tumor volume (Figure 6A). Real-time PCR analysis of tumor samples found that the cells in the experimental group expressed the early neural marker molecule DCX (Figure 6B), indicating that the glioma cells in the animal body were induced to become neurons, resulting in the inhibition of tumor growth.

Example 7 Application of other delivery systems in delivery of functional fragments of transcription factors

In addition to the lentivirus vector and adeno-associated virus vector described in the above embodiment, other types of delivery systems can also achieve similar functions. In this embodiment, the adenovirus vector type 5 expressing NeuroD1 and Brn2 can also inhibit the growth of human glioma U87 BALB/CA-nu mice heterotopic implantation model tumor cells.

Because adeno-associated virus can not replicate independently in vivo, we designed adenovirus type 5 vector to express reprogramming factors efficiently and rapidly. With the specific amplification of adenovirus type 5 in tumor cells, we can achieve the effect of in vivo trans-differentiation therapy to control the recurrence of glioma.

On the vector template of Adeno-Cas9 (Addgene, # 64072), the CDS (SEQ ID No.: 7) fragment derived from human NeuroD1 (SEQ ID No.: 3) and human Brn2 gene was constructed on the vector through the Age I/Spe I double restriction site to obtain Ad5-hNeuroD1-P2A-hBrn2 (Ad5-AN). P2A is a self-cleaving polypeptide, which can achieve high co-expression of hNeuroD1 and hBrn2.

We used the heterotopic inoculation model of human glioma U87 BALB/CA-nu mice. At the logarithmic growth stage, the U87 human glioma cells in culture were inoculated into the armpit of nude mice, and when the tumor grew to about 100mm$^3$, the nude mice with appropriate tumor mass were randomly divided into groups, and the drug was administered after grouping. The control group was PBS group, and the dose of Ad5-AN-low group was $3\times10^8$ PFU Ad5-vector-high group dose $1\times10^9$ PFU, administered once every two days, for five consecutive times. The volume of tumor was measured and calculated every 3 days, and the animals were killed in the later stage, and the tumor was weighed for biochemical and molecular detection.

The results showed that compared with the control PBS group, the tumor volume of Ad5-AN-low group decreased by 32.25%, and that of Ad5-AN-high group decreased by 67.49% (Figure 7A). It can be seen that the reprogramming of glioma significantly reduces the growth of tumor cells. HE staining also found that the growth of glioma was inhibited (Fig. 7B). These results suggest that reprogramming factor-mediated glioma trans-differentiation in vivo leads to tumor cell growth inhibition.

We also use exosomes (GBM-Exo) derived from mesenchymal cells or glioblastoma cells. The exosomes were extracted from the supernatant of cell culture by density gradient centrifugation and molecular exclusion separation, and the expression of exosome-marker protein CD63 was identified by Western blot, the shape characteristics and particle size of exosomes were detected by transmission electron microscopy and dynamic light scattering, and the concentration of exosomes was detected by BCA protein quantitative method.

For glioblastoma, Ascl1-mRNA (NCBI Reference Sequence: NM_004316.4) or other transcription factor combinations described in the present invention are introduced into the exocrine body through endogenous expression or exogenous introduction. Exocrine drugs derived from glioblastoma will specifically infect human glioblastoma cell lines U251 and U87. In the logarithmic growth phase of cells, it can be observed that the efficiency of exocrine drugs inducing human glioblastoma cell lines U251 and U87 into neurons can change with the concentration gradient of exocrine drugs, and the rate of related tumor cell proliferation is also proportional to the efficiency of neuron induction.

All documents mentioned in the present invention are cited as references in this application, just as each document is cited separately as a reference. In addition, it should be understood that after reading the above lectures of the invention, those skilled in the art can make various changes or modifications to the invention, and these equivalent forms also fall within the scope of the claims attached to the application.

## Claims

1. A group of functional fragments that can synergistically promote the trans-differentiation of glial cells, wherein the functional fragments contain at least one functional fragment that can promote the expression of transcription factors, and the functional fragments are selected from those that can promote the expression of Ascl1, NeuroD1, Brn2, Ngn2, Gsx1, Tbr1, Dlx2, Ptf1a, Pax6 and/or Otx2 transcription factors.

2. The group of functional fragments that can synergistically promote the trans-differentiation of glial cells of claim 1, wherein the Ascl1 is an enhanced Ascl1, and its amino acid sequence is shown in SEQ ID No: 41.

3. The group of functional fragments that can synergistically promote the trans-differentiation of glial cells of claim 1, wherein the functional fragments contain at least two functional fragments that promote the expression of transcription factors, and the functional fragments are selected from the those that can promote the expression of NeuroD1, Brn2, Ascl1, Ngn2, Gsx1, Tbr1, Dlx2, Ptf1a, Pax6 and/or Otx2 transcription factors;
   Preferably, the functional fragments contain at least a functional fragment that can promote the expression of any one of the Ascl1 and Ngn2 transcription factors.

4. The group of functional fragments that can synergistically promote the trans-differentiation of glial cells of claim 1, wherein the functional fragments at least contain functional fragments that promote the expression of NeuroD1 and Brn2 transcription factors, or functional fragments that promote the expression of Gsx1 and Tbr1 transcription factors, or functional fragments that promote the expression of Dlx2 and Ptf1a transcription factors, or functional fragments that promote the expression of Pax6 and Otx2 transcription factors;
   Preferably, the functional fragments also contain functional fragments that promote the expression of Ascl1 or Ngn2 transcription factors.

5. The group of functional fragments that can synergistically promote the trans-differentiation of glial cells of claim 1, wherein the functional fragments are selected from the polynucleotides encoding the transcription factor, or the functional proteins and peptides after the translation of the polynucleotides, or the small molecule drugs, macromolecular drugs, nucleic acid drugs that promote the expression of the transcription factor, or the polynucleotides or functional proteins, peptides, small molecule drugs, or macromolecular drugs, nucleic acid drugs that are located upstream of the transcription factor and regulate the up-regulation of the expression of the transcription factor.

6. The group of functional fragments that can synergistically promote the trans-differentiation of glial cells of claim 4, wherein the functional fragments are selected from a transcription factor functional protein with sequence homology of not less than 85% with SEQ ID NO.: 1, 2, 5, 6, 9, 10, 13, 14, 17, 18, 21, 22, 25, 26, 29, 30, 33, 34, 37, 38 and/or 41, or from a polynucleotide encoding a transcription factor with sequence homology of not less than 75% with SEQ ID NO.: 3, 4, 7, 8, 11, 12, 15, 16, 19, 20, 23, 24, 27, 28, 31, 32, 35, 36, 39 and/or 40;

   Preferably, the functional fragments are selected from a transcription factor functional protein whose sequence homology with SEQ ID NO.: 1, 2, 5, 6, 9, 10, 13, 14, 17, 18, 21, 22, 25, 26, 29, 30, 33, 34, 37, 38 and/or 41 is not less than 95%, Or polynucleotides encoding transcription factors with sequence homology of not less than 85% with SEQ ID NO.: 3, 4, 7, 8, 11, 12, 15, 16, 19, 20, 23, 24, 27, 28, 31, 32, 35, 36, 39 and/or 40;
   More preferably, the functional fragments are selected from a transcription factor functional protein with sequence homology of not less than 99% with SEQ ID NO.: 1, 2, 5, 6, 9, 10, 13, 14, 17, 18, 21, 22, 25, 26, 29, 30, 33, 34, 37, 38 and/or 41, Or polynucleotides encoding transcription factors with sequence homology of not less than 95% with SEQ ID NO.: 3, 4, 7, 8, 11, 12, 15, 16, 19, 20, 23, 24, 27, 28, 31, 32, 35, 36, 39 and/or 40.

7. The group of functional fragments that can synergistically promote the trans-differentiation of glial cells of claim 1, wherein the functional fragments that promote the expression of transcription factors can promote the expression of related transcription factors in glial cells by directly contacting with the glial cells or introducing them into the delivery system, and display the characteristics of functional nerve cells or neuro-like cells;

Preferably, the glial cells are selected from astrocytes, NG2 glial cells, oligodendrocytes, microglial cells, or glial cells in the injured state, and tumor cells derived from glial cells; or

The delivery system is selected from an expression vector containing functional fragments that promote the expression of transcription factors, nanoparticles wrapped with functional fragments that promote the expression of transcription factors, exosomes wrapped with functional fragments that promote the expression of transcription factors, viral vectors or cell vectors wrapped with functional fragments that promote the expression of transcription factors, and targeted effectors that contain functional fragments that promote the expression of transcription factors; or

The delivery system also contains glial cell-specific promoters or expression regulatory elements.

8. The group of functional fragments that can synergistically promote the trans-differentiation of glial cells of claim 7, wherein the expression system of the functional fragments that promote the expression of transcription factors is constructed on the same expression vector or expressed separately using different expression vectors, wherein:

(1) When there are any two functional fragments that promote the expression of transcription factors, the molar concentration ratio of the expression of the two transcription factors is 4:1 to 1:4; Preferably, the molar concentration ratio of the expression amount of the two transcription factors is 2:1 to 1:2; More preferably, the molar concentration ratio of the expression of the two transcription factors is 1:1; or

(2) When there are two or more functional fragments promoting the expression of transcription factors, and one of the functional fragments promoting the expression of transcription factors is the functional fragment promoting the expression of Ascl1 or Ngn2 transcription factors, the molar concentration ratio of the expression of Ascl1 or Ngn2 is not less than 20%, Preferably, the molar concentration ratio of the expression of Ascl1 or Ngn2 is not less than 33%; More preferably, the molar concentration ratio of the expression amount of Ascl1 or Ngn2 is not less than 50%.

9. The group of functional fragments that can synergistically promote the trans-differentiation of glial cells of claim 8, wherein the functional fragments that promote the expression of transcription factors are selected from:

(1) Combination of functional fragments that promote the expression of transcription factors of NeuroD1 and Brn2;
(2) Combination of functional fragments that promote the expression of transcription factors of Gsx1 and Tbr1;
(3) Combination of functional fragments that promote the expression of transcription factors of Dlx2 and Ptfla;
(4) Combination of functional fragments that promote the expression of transcription factors of Pax6 and Otx2;
(5) Combination of functional fragments that promote the expression of Ascl1 and any other transcription factor, and the other transcription factor is selected from NeuroD1, Brn2, Ngn2, Gsx1, Tbr1, Dlx2, Ptf1a, Pax6 or Otx2;
(6) Combination of functional fragments that promote the expression of Ngn2 and any other transcription factor, and the other transcription factor is selected from NeuroD1, Brn2, Ascl1, Gsx1, Tbr1, Dlx2, Ptf1a, Pax6 or Otx2.

10. The group of functional fragments that can synergistically promote the trans-differentiation of glial cells of claim 8, wherein the functional fragments that promote the expression of transcription factors are selected from:

(1) Combination of functional fragments that promote the expression of transcription factors of Ascl1, NeuroD1 and Brn2;
(2) Combination of functional fragments that promote the expression of transcription factors of Ascl1, Gsx1 and Tbr1;
(3) Combination of functional fragments that promote the expression of transcription factors of Ascl1, Dlx2 and Ptfla;
(4) Combination of functional fragments that promote the expression of transcription factors of Ascl1, Pax6 and Otx2;
(5) Combination of functional fragments that promote the expression of transcription factors of Ngn2, NeuroD1 and Brn2;
(6) Combination of functional fragments that promote the expression of transcription factors of Ngn2, Gsx1 and Tbr1;
(7) Combination of functional fragments that promote the expression of transcription factors of Ngn2, Dlx2 and Ptfla;

(8) Combination of functional fragments that promote the expression of transcription factors of Ngn2, Pax6 and Otx2;

Among them, except Ascl1 or Ngn2, the molar concentration ratio of the expression of the remaining two transcription factors is 4:1 to 1:4; Preferably, the molar concentration ratio of the expression amount of the remaining two transcription factors is 2:1 to 1:2; More preferably, the molar concentration ratio of the expression of the remaining two transcription factors is about 1:1.

11. The group of functional fragments that can synergistically promote the trans-differentiation of glial cells of claim 1, wherein the trans-differentiation refers to the trans-differentiation or reprogramming of glial cells into functional neurons.

12. The group of functional fragments that can synergistically promote the trans-differentiation of glial cells of claim 1, wherein the combination of the functional fragments is selected from the following group:

(Z1) NeuroD1+Brn2;
(Z2) Ascl1+Ngn2;
(Z3) Ngn2+NeuroD1;
(Z4) Gsx1+Tbr1;
(Z5) Dlx2+Ptfla;
(Z6) Pax6+Otx2;
(Zn) The combination of (Z1) to (Z6) above.

13. The group of functional fragments that can synergistically promote the trans-differentiation of glial cells of claim 1, wherein the combination of the functional fragments is selected from the following group: NeuroD1+Brn2; Gsx1+Tbr1; Dlx2+Ptfla; Pax6+Otx2; Or a combination thereof.

14. A method for promoting the trans-differentiation and reprogramming of glial cells into functional neurons or neuron-like cells, comprising the following steps: contacting any of the functional fragments described in claims 1-13 that can synergistically promote the trans-differentiation of glial cells with glial cells, so as to enable the trans-differentiation and reprogramming of glial cells into functional neurons or neuron-like cells.

15. An artificial reprogrammed neuron or neuron-like, wherein the neuron or neuron-like is prepared by the method described in claim 14.

16. The application of the functional fragments that can synergistically promote the trans-differentiation of glial cells as described in any of claims 1-13 in the preparation of drugs for nervous system diseases, wherein the nervous system diseases are nervous system diseases, injuries or gliomas derived from glial cells.

17. A pharmaceutical composition comprising:
A pharmaceutical composition comprising:

(A) A combination of functional fragments that promote the expression of transcription factors, or functional neurons or neuro-like elements obtained by processing glial cell trans-differentiation and reprogramming with the combination;

Wherein, the combination contains Ascl1 or enhanced Ascl1; Or Ngn2; or
The combination is selected from the following group:

(Z1) NeuroD1+Brn2;
(Z2) Ascl1+Ngn2;
(Z3) Ngn2+NeuroD1;
(Z4) Gsx1+Tbr1;
(Z5) Dlx2+Ptfla;
(Z6) Pax6+Otx2; and
(Zn) the combination of (Z1) to (Z6) above;

(B) Pharmaceutical acceptable excipients.

**18.** The pharmaceutical composition of claim 17, wherein the combination is the combination of enhanced Ascl1 and at least one selected from the following groups: NeuroD1, Brn2, Ngn2, Gsx1, Tbr1, Dlx2, Ptf1a, Pax6, Otx2.

**19.** The pharmaceutical composition of claim 17, wherein the combination is the combination of Ascl1 and at least one selected from the following groups: NeuroD1, Brn2, Ngn2, Gsx1, Tbr1, Dlx2, Ptf1a, Pax6, Otx2.

**20.** The pharmaceutical composition of claim 17, wherein the combination is the combination of Ngn2 and at least one selected from the following groups: NeuroD1, Brn2, Ngn2, Gsx1, Tbr1, Dlx2, Ptf1a, Pax6, Otx2.

Fig. 1

Fig. 2

Fig. 3

Fig. 4

Fig. 5

Fig. 6

Fig. 7

## INTERNATIONAL SEARCH REPORT

| | International application No. |
|---|---|
| | **PCT/CN2021/117302** |

**A. CLASSIFICATION OF SUBJECT MATTER**

C12N 15/12(2006.01)i; A61K 38/19(2006.01)i; A61K 49/14(2006.01)i; C12N 15/85(2006.01)i; C12N 15/87(2006.01)i

According to International Patent Classification (IPC) or to both national classification and IPC

**B. FIELDS SEARCHED**

Minimum documentation searched (classification system followed by classification symbols)

C12N A61K

Documentation searched other than minimum documentation to the extent that such documents are included in the fields searched

Electronic data base consulted during the international search (name of data base and, where practicable, search terms used)

CNABS, SIPOABS, DWPI, CNKI, baidu, patentics, NCBI: 再康医药科技（上海）有限公司, 王亚里, 刘月光, 陈如雷, 协同促进胶质细胞转分化, 胶质细胞, 转分化, 促进转录因子表达, 转录因子, 神经系统损伤, 脑肿瘤, 神经胶质瘤, 胶质瘤, 胶质细胞转分化为神经元, 星形胶质细胞转分化神经元, 增强型Ascl1, Ascl1, Sox9, Achaete-scute homolog 1, achaete-scute complex homolog 1, glial, differentiat+, trans-differentiat+, reprogram+, interconversion, neuroglial, astrocyte, oligodendrocyte, neuron, NeuroD1, neuronal differentiation 1, Brn2, Ngn2, Gsx1, Tbr1, Dlx2, Ptf1a, Pax6, Paired box protein 6, Otx2, orthodentic le homeobox 2, NeuroD1+Brn2, Ascl1+Ngn2, Ngn2+NeuroD1, Gsx1+Tbr1, Dlx2+Ptf1a, Pax6+Otx2, NOD-scid.

**C. DOCUMENTS CONSIDERED TO BE RELEVANT**

| Category* | Citation of document, with indication, where appropriate, of the relevant passages | Relevant to claim No. |
|---|---|---|
| X | CN 102753690 A (VIVOSCRIPT, INC.) 24 October 2012 (2012-10-24)<br>claims 1-36, and description, abstract, figure 18 | 1, 3-5, 7-20 |
| X | WO 2020041142 A1 (RUTGERS, THE STATE UNIVERSITY OF NEW JERSEY) 27 February 2020 (2020-02-27)<br>claim 1, and description, figure 39 | 1, 5, 7, 11, 14-16 |
| X | CN 107435050 A (XIANG, Mengqing et al.) 05 December 2017 (2017-12-05)<br>description, specific embodiments | 1, 5, 7, 11, 14-16 |
| X | WO 2010144696 A1 (BURNHAM INSTITUTE FOR MEDICAL RESEARCH) 16 December 2010 (2010-12-16)<br>claims 1 and 78-80 | 1, 5, 7, 11, 14-16 |
| Y | CN 102753690 A (VIVOSCRIPT, INC.) 24 October 2012 (2012-10-24)<br>claims 1-36, abstract, and figure 18 | 1-20 |

☑ Further documents are listed in the continuation of Box C.    ☑ See patent family annex.

| | |
|---|---|
| *   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **19 November 2021** | **25 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/117302** |

**C.    DOCUMENTS CONSIDERED TO BE RELEVANT**

| Category* | Citation of document, with indication, where appropriate, of the relevant passages | Relevant to claim No. |
| --- | --- | --- |
| Y | WO 2020041142 A1 (RUTGERS, THE STATE UNIVERSITY OF NEW JERSEY) 27 February 2020 (2020-02-27)<br>        claim 1, and description, figure 39 | 1-20 |
| Y | CN 107435050 A (XIANG, Mengqing et al.) 05 December 2017 (2017-12-05)<br>        description, specific embodiments | 1-20 |
| Y | WO 2010144696 A1 (BURNHAM INSTITUTE FOR MEDICAL RESEARCH) 16 December 2010 (2010-12-16)<br>        claims 1 and 78-80 | 1-20 |
| Y | NAGASE, T. et al. "achaete-scute complex homolog 1, partial [synthetic construct], GenBank: BAG73959.1, 237aa linear."<br>*NCIB genbank.*, 26 July 2016 (2016-07-26),<br>        p. 1 | 2 |
| Y | LUCK, K. et al. "neurogenic differentiation factor 1 [Homo sapiens], NCBI Reference Sequence: NP_002491.3, 356aa linear."<br>*NCIB genbank.*, 29 August 2020 (2020-08-29),<br>        pp. 1-3 | 6 |
| Y | URNESS, L. D. et al. "neurogenic differentiation factor 1 [Mus musculus], NCBI Reference Sequence: NP_035024.1, 357aa linear."<br>*NCIB genbank.*, 29 August 2020 (2020-08-29),<br>        pp. 1-3 | 6 |
| Y | NCBI Genbank. "POU domain, class 3, transcription factor 2 [Pan troglodytes], NCBI Reference Sequence: NP_001289356.1, 443aa linear."<br>*NCBI GenBank.*, 26 June 2020 (2020-06-26),<br>        pp. 1 and 2 | 6 |
| Y | NASU, M. et al. "POU domain, class 3, transcription factor 2 [Mus musculus], NCBI Reference Sequence: NP_032925.1, 445aa linear."<br>*NCIB genbank.*, 06 September 2020 (2020-09-06),<br>        pp. 1 and 2 | 6 |
| A | CN 109385404 A (GUIDON PHARMACEUTICS, INC.) 26 February 2019 (2019-02-26)<br>        entire document | 1-20 |
| A | WO 2011091048 A1 (THE BOARD OF TRUSTEES OF THE LELAND STANFORD JUNIOR UNIVERSITY) 28 July 2011 (2011-07-28)<br>        entire document | 1-20 |
| A | US 2020002679 A1 (KCELLBIO BANKING CO., LTD.) 02 January 2020 (2020-01-02)<br>        entire document | 1-20 |
| A | US 2020018746 A1 (THE BROAD INSTITUE, INC. et al.) 16 January 2020 (2020-01-16)<br>        entire document | 1-20 |
| A | EP 3418378 A1 (HI-STEM GGMBH IM DEUTSCHEN KREBSFORSCHUNGSZENTRUM DKFZ) 26 December 2018 (2018-12-26)<br>        entire document | 1-20 |
| A | WO 2010053522 A3 (THE REGENTS OF THE UNIVERSITY OF CALIFORNIA) 16 September 2010 (2010-09-16)<br>        entire document | 1-20 |
| A | WO 2015114059 A1 (HELMHOLTZ ZENTRUM MüNCHEN-DEUTSCHES FORSCHUNGSZENTRUM FüR GESUNDHEIT UND UMWELTGMBH) 06 August 2015 (2015-08-06)<br>        entire document | 1-20 |
| A | WO 2017081033 A1 (UNIVERSITY DE NICE SOPHIA ANTIPOLI et al.) 18 May 2017 (2017-05-18)<br>        entire document | 1-20 |

Form PCT/ISA/210 (second sheet) (January 2015)

**INTERNATIONAL SEARCH REPORT**

| International application No. |
| --- |
| **PCT/CN2021/117302** |

## C.    DOCUMENTS CONSIDERED TO BE RELEVANT

| Category* | Citation of document, with indication, where appropriate, of the relevant passages | Relevant to claim No. |
| --- | --- | --- |
| A | VUE, T.Y. et al. "Ascl1 controls the number and distribution of astrocytes and oligodendrocytes in the gray matter and white matter of the spinal cord." *Development.*, Vol. 141, No. 19, 31 October 2014 (2014-10-31), pp. 3721-3731 | 1-20 |
| A | DENNIS, D. J. et al. "Neurog2 and ascl1 together regulate a postmitotic derepression circuit to govern laminar fate specification in the murine neocortex." *Proc Natl Acad Sci.*, Vol. 114, No. 25, 05 June 2017 (2017-06-05), pp. E4934-E4943 | 1-20 |

Form PCT/ISA/210 (second sheet) (January 2015)

**INTERNATIONAL SEARCH REPORT**

International application No.

**PCT/CN2021/117302**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT

International application No.

**PCT/CN2021/117302**

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **14** （部分）
   because they relate to subject matter not required to be searched by this Authority, namely:

   [1]   The technical solution of claim 14 (in part) relates to a "method for treating diseases", belonging to the subject matter as defined in Rule 39(iv); however, a search is made on the basis of a "use in the preparation of a drug".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**INTERNATIONAL SEARCH REPORT**
Information on patent family members

International application No.

**PCT/CN2021/117302**

| Patent document cited in search report | | | Publication date (day/month/year) | Patent family member(s) | | | Publication date (day/month/year) |
|---|---|---|---|---|---|---|---|
| CN | 102753690 | A | 24 October 2012 | US | 2012301446 | A1 | 29 November 2012 |
| | | | | WO | 2011097181 | A3 | 23 February 2012 |
| | | | | WO | 2011097181 | A2 | 11 August 2011 |
| | | | | JP | 2013518587 | A | 23 May 2013 |
| | | | | EP | 2531599 | A4 | 08 January 2014 |
| | | | | EP | 2531599 | A2 | 12 December 2012 |
| WO | 2020041142 | A1 | 27 February 2020 | CA | 3110309 | A1 | 27 February 2020 |
| CN | 107435050 | A | 05 December 2017 | CN | 107435050 | B | 09 February 2021 |
| WO | 2010144696 | A1 | 16 December 2010 | US | 2011002897 | A1 | 06 January 2011 |
| CN | 109385404 | A | 26 February 2019 | | None | | |
| WO | 2011091048 | A1 | 28 July 2011 | US | 2017369840 | A1 | 28 December 2017 |
| | | | | US | 2015284681 | A1 | 08 October 2015 |
| | | | | US | 2013022583 | A1 | 24 January 2013 |
| | | | | US | 2018057789 | A1 | 01 March 2018 |
| | | | | US | 9822338 | B2 | 21 November 2017 |
| | | | | US | 9057053 | B2 | 16 June 2015 |
| US | 2020002679 | A1 | 02 January 2020 | | None | | |
| US | 2020018746 | A1 | 16 January 2020 | | None | | |
| EP | 3418378 | A1 | 26 December 2018 | CA | 3067447 | A1 | 27 December 2018 |
| | | | | WO | 2018234491 | A1 | 27 December 2018 |
| | | | | US | 2020140812 | A1 | 07 May 2020 |
| | | | | JP | 2020524504 | A | 20 August 2020 |
| | | | | EP | 3642333 | A1 | 29 April 2020 |
| WO | 2010053522 | A3 | 16 September 2010 | WO | 2010053522 | A2 | 14 May 2010 |
| WO | 2015114059 | A1 | 06 August 2015 | US | 2017002316 | A1 | 05 January 2017 |
| | | | | US | 10752881 | B2 | 25 August 2020 |
| | | | | EP | 3099786 | A1 | 07 December 2016 |
| | | | | JP | 2020039345 | A | 19 March 2020 |
| | | | | JP | 2017509322 | A | 06 April 2017 |
| WO | 2017081033 | A1 | 18 May 2017 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Pharmaceutics,* 2019, vol. 11 (11), 580
- **SAMBROOK et al.** Molecular Cloning: Laboratory Manual. Cold Spring Harbor Laboratory Press, 1989
- **VIERBUCHEN, T. et al.** Direct conversion of fibroblasts to functional neurons by defined factors. *Nature,* 2010, vol. 463, 1035-1041
- *Proc Natl Acad Sci U S A,* vol. 93, 11382-11388
- **TISCORNIA, G. ; SINGER, O. ; VERMA, I. M.** Production and purification of lentivirus vectors. *Nat. Protocol.,* 2006, vol. 1, 241-245
- **MCDONOUGH A ; MONTERUBIO A ; ARIZONA J et al.** Calibrated Forceps Model of Spinal Cord Compression Injury. *Jove-Journal of Visualized Experiments,* 2015
- *J Neurotrauma,* 2006, vol. 23 (5), 635-59